# EUROPEAN PATENT SPECIFICATION

(11) **EP 4 294 454 B1**
(45) Date of publication and mention of the grant of the patent: **15.04.2026**
(21) Application number: 22755541.4
(22) Date of filing: 16.02.2022
(51) Int. Cl.: A61K 38/04, A61K 47/68, C07K 16/28, A61P 31/12, A61K 38/03

(54) **ANTI-VIRAL PEPTIDES AND COMPOSITIONS AND METHODS OF USE THEREOF**
ANTIVIRALE PEPTIDE UND ZUSAMMENSETZUNGEN UND VERFAHREN ZUR VERWENDUNG DAVON
PEPTIDES ANTI-VIRAUX ET COMPOSITIONS ET PROCÉDÉS D'UTILISATION ASSOCIÉS

(30) Priority: 17.02.2021 US 202163150110 P; 17.02.2021 US 202163150141 P
(43) Date of publication of application: 27.12.2023
(73) Proprietor: Versitech Limited, Hong Kong (CN); Centre for Virology, Vaccinology and Therapeutics Limited, Shatin Hong Kong (HK)
(72) Inventor: ZHAO, Hanjun, Hong Kong (CN); YUEN, Kwok-Yung, Hong Kong (CN)
(74) Representative: HGF
(86) International application number: PCT/CN2022/076453
(87) International publication number: WO 2022/174768

(56) References cited:
- WO-A1-03/041710
- WO-A1-2020/047333
- WO-A1-2020/231855
- WO-A1-2021/015437
- WANG XI, CAO RUIYUAN, ZHANG HUANYU, LIU JIA, XU MINGYUE, HU HENGRUI, LI YUFENG, ZHAO LEI, LI WEI, SUN XIULIAN, YANG XINGLOU, SHI Z: "The anti-influenza virus drug, arbidol is an efficient inhibitor of SARS-CoV-2 in vitro", CELL DISCOVERY, vol. 6, no. 1, 1 December 2020 (2020-12-01), XP055961451, DOI: 10.1038/s41421-020-0169-8
- HUANG MINGXING, LI MAN, XIAO FEI, PANG PENGFEI, LIANG JIABI, TANG TIANTIAN, LIU SHAOXUAN, CHEN BINGHUI, SHU JINGXIAN, YOU YINGYING: "Preliminary evidence from a multicenter prospective observational study of the safety and efficacy of chloroquine for the treatment of COVID-19", NATIONAL SCIENCE REVIEW, vol. 7, no. 9, 12 September 2020 (2020-09-12), pages 1428 - 1436, XP055772426, ISSN: 2095-5138, DOI: 10.1093/nsr/nwaa113
- MAISONOASSE, P. ET AL.: "Hydroxychloroquine use against SARS-CoV-2 infection in non-human primates", NATURE, vol. 585, 22 July 2020 (2020-07-22), pages 584 - 587, XP037253993, DOI: 10.1038/s41586-020-2558-4

## Description

### FIELD OF THE INVENTION

The invention is generally directed to broad spectrum antiviral peptides and methods of use thereof in treating antiviral infections.

### BACKGROUND OF THE INVENTION

Before COVID-19, limited attention was directed to the discovery of anti-coronavirus drugs, even after the SARS-CoV outbreak in 2003. Influenza viruses which cause pandemic and seasonal outbreaks have repeatedly overwhelmed healthcare institutions and affected socioeconomic activities. The suboptimal effectiveness of currently available anti-influenza drugs against certain strains was evidenced by the high mortality rates (>30%) of Influenza A(H5N1) and A(H7N9) viruses in patients (1, 2). Drug resistant viruses can emerge quickly in patients while on treatment with specific anti-influenza drugs such as oseltamivir and baloxavir (3, 4). Moreover, resistant viruses against anti-influenza neutralizing monoclonal antibody could be identified after extensive virus passaging (5, 6). Besides the endless influenza outbreaks, the novel SARS-CoV-2 has emerged and disseminated globally since early 2020. Together with the circulating seasonal influenza virus, SARS-CoV-2 may cause co-infection with increased severity during the influenza season (7-9). These circulating influenza virus, coronavirus and resistant virus mutants revealed a poor capability in responding to threats of emerging/reemerging viruses with the currently available antivirals (1, 2).

Antiviral peptides with broad-spectrum antiviral activities against influenza virus and/or coronavirus have shown promising prospects with little metabolic toxicity and low possibility of inducing drug-resistant viruses (10-16). Due to the lack of proofreading activity of RNA polymerases in RNA viruses, SARS-CoV-2 mutants were not infrequently found in patients during the galloping pandemic (17). Similarly, drug-resistant viral mutants emerged during treatment by specific antivirals, especially the small molecular compounds including neuraminidase inhibitors, M2 and polymerase inhibitors (3, 4). Defensins, naturally existing in almost all multicellular plants and animals, have been shown to have broad antiviral activities against influenza virus, coronavirus and other viruses (18, 19). Moreover, defensin-induced resistant viruses have not been reported which is consistent with the finding that defensin-derived peptide, P9R, did not induce drug-resistant virus even after extensive virus passaging in the presence of P9R (16). Chloroquine with broad-spectrum antiviral activities targeting host factors was effective inhibiting pH-dependent viruses in vitro and in vivo (36-38), but not effective in vivo in some other studies with unclear reason (28, 29, 39). The broad-spectrum antiviral activity and low chance of inducing drug-resistant viruses make the defensin-derived peptide and similar host-targeting antivirals are promising candidates for drug development.

Thus, there is an urgent need for broad-spectrum antivirals for combating emerging and re-emerging virus outbreaks, such as the SARS-CoV-2, before the virus is identified or a specific antiviral drug is available. Broad-spectrum agents inhibiting both influenza virus and coronavirus with new antiviral mechanisms and showing a low probability of inducing drug-resistance are urgently needed for circumventing emerging viruses.

WO 2020/231855 discloses a pharmaceutical compositions comprising an IL-15 agonist or derivative thereof, such as nogapendekin alfa-inbakicept (NAI).

WO2021/015437 discloses a composition comprising an reactive oxygen species production inhibitor such as 4-phenylbutyrate (4-PBA) for preventing or treating a viral infection disease.

### SUMMARY OF THE INVENTION

It is an object of the present invention to provide broad spectrum antiviral agents.

It is also an object of the present invention to provide compositions of broad spectrum antiviral agents.

It is still an object of the present invention to provide methods for treating viral infections in a subject in need thereof.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

A broad-spectrum antiviral peptide, P16, has been discovered which significantly inhibits A(H1N1), A(H3N2), influenza B virus and SARS-CoV-2 infections. Mechanistic studies demonstrate that P16 has dual-functional activities: P16 inhibits HA fusion of influenza viruses by direct binding and antagonizes endosomal acidification to suppress influenza virus and SARS-CoV-2 entry through the endocytic pathway. *In vivo* studies indicate that P16 improved survival of A(H1N1)-challenged mice and inhibited viral replication in mouse lungs. Thus, antiviral agents, compositions containing the antiviral agents and the antivial agents for use in methods described herein are provided.

In particular, disclosed are antiviral agents and antiviral compositions. In some forms, the antiviral agents and compositions inhibit antiviral replication in cells. In some forms, the antiviral agents and compositions inhibit viral entry into cells. In some forms, the antiviral agents and compositions inhibit viral entry into cells and antiviral replication in cells.

The antiviral agent includes one or more copies of P16 containing the amino acid sequence of RGAHIKGRWKSRCHRF (SEQ ID NO:1).

In a preferred form, the antiviral agent consists of P16 peptide having the amino acid sequence of SEQ ID NO:1.

The P16 can have a net positive charge. For example, in some forms, the P16 has a net positive charge of at least 1, 2, 3, 4, 5 or 6. Preferably, the P16 has a net positive charge of at least 6. In some preferred forms, the P16 has a net positive charge of about 6.1.

The antiviral agent can exhibit a number of antiviral properties. For example, in some forms, upon contact with a virus, the P16 binds to the virus, reduces or prevents fusion of the virus with target cells, inhibits endosomal acidification of target cells, reduces or prevents cell entry of the virus, reduces or prevents infection of target cells by the virus, reduces or prevents replication of the virus, or combinations thereof. The contacting between the antiviral agent can occur *in vitro* or in a subject *in vivo.*

Antiviral compositions are also provided. In some forms, the antiviral compositions include any one or more of the disclosed antiviral agents and a pharmaceutically acceptable carrier. In some forms the antiviral compositions include a therapeutically effective amount of any one or more of the disclosed antiviral agents and a pharmaceutically acceptable carrier.

The compositions can be in a unit dosage form. In some forms, the unit dosage form is selected from a table or capsule. In some forms, the composition is in a form suitable for intranasal or pulmonary delivery. In some forms, the unit dosage form is an injectable, where the composition further comprises a pharmaceutically acceptable carrier for injection to a human.
**Also disclosed are the antiviral agents for use in methods of treating viral infection in a subject in need** thereof. In some forms, the method includes administering to the subject an effective amount of any of the disclosed antiviral agents or any of the disclosed antiviral compositions.

In some forms, the infection is caused by a respiratory virus. In some forms, the infection is caused by a pH-dependent virus that requires endosomal acidification for virus-host membrane fusion. In some forms, the infection is caused by zika virus, enterovirus-A7, ebola virus, influenza virus (e.g., influenza A virus, influenza B virus), SARS-CoV-2, SARS-CoV, MERS-CoV, and the non-enveloped rhinovirus. Exemplary influenza viruses include, A(H7N9), influenza A(H7N7), influenza A(H5N1), influenza A(H1N1) (e.g., the A(H1N1)pdm09 virus) and influenza A(H3N2).

In some forms, the composition is administered parenterally or orally. In some forms, the composition is administered intranasally, or by pulmonary administration.

In some forms, the method further includes administering an effective amount of chloroquine to the subject. Preferably, the chloroquine is not administered systemically (e.g., orally). In some forms, the chloroquine is administered intranasally or by pulmonary administration. Typically, the chloroquine can be in a inhalable form or formulation. In some forms, the chloroquine is administered via intranasal inoculation or atomization inhalation

In some forms, the antiviral agent or composition is administered in an effective amount to reduce one or more symptoms of disease, disorder, or illness associated with virus. Suitable symptoms include fever, congestion in the nasal sinuses and/or lungs, runny or stuffy nose, cough, sneezing, sore throat, body aches, fatigue, shortness of breath, chest tightness, wheezing when exhaling, chills, muscle aches, headache, diarrhea, tiredness, nausea, vomiting, and combinations thereof. In some forms, the antiviral agent or composition is administered in an effective amount to reduce or prevent viral replication in the subject.

In any of the foregoing methods the subject is preferably human.

### BRIEF DESCRIPTION OF THE DRAWINGS

**The invention is defined in the appended claims. Any of the following figures that do not fall within the scope of the claims are provided for illustrative or comparative purposes only.**

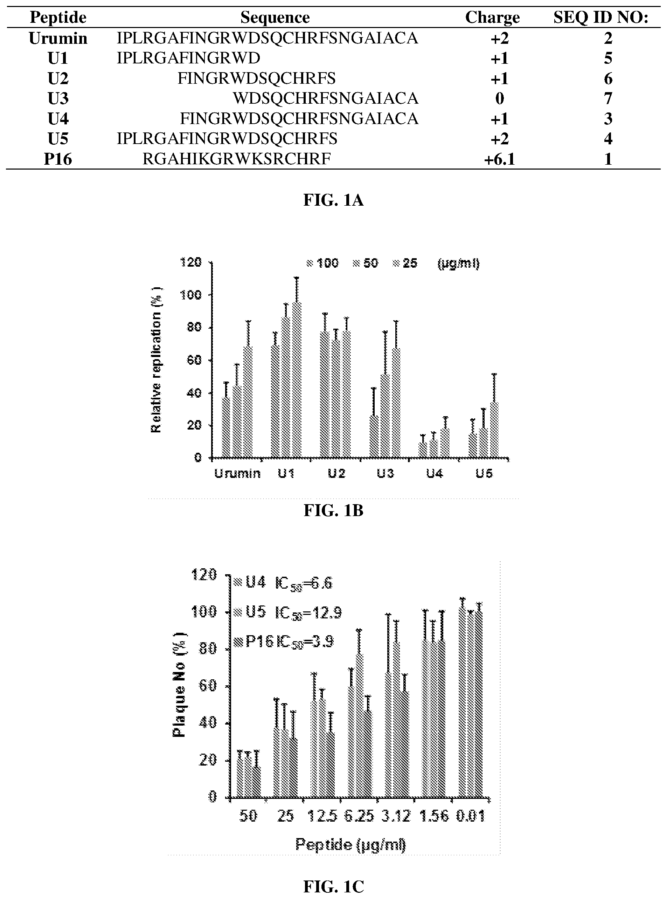

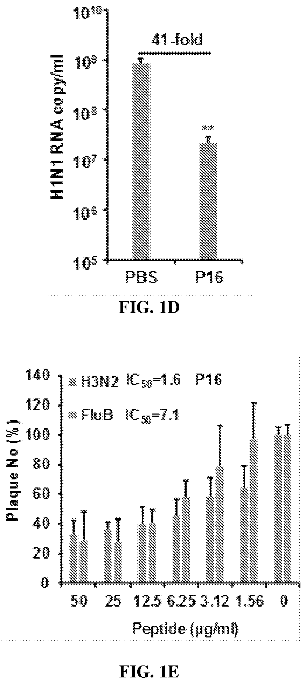

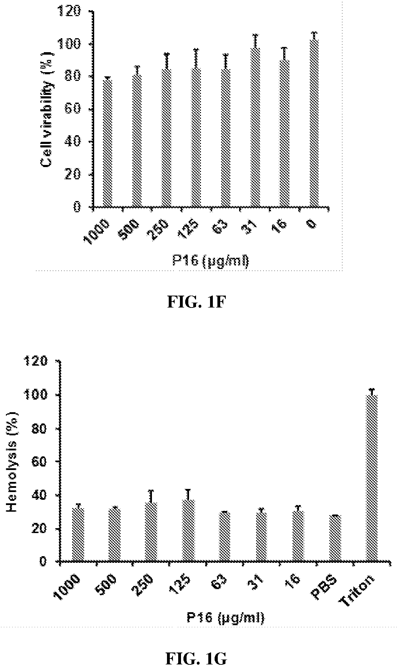

**Figures 1A-1G** show the identification and characterization of short basic peptides against influenza A and B viruses. **Figure 1A** shows the peptide sequences and their charges calculated by PepCalc of InnovaGen relative to pH 7.0. **Figure 1B** is a bar graph showing the antiviral activity of the indicated peptides at various concentrations against A(H1N1) virus (n=3). Virus was pretreated by peptides with the indicated concentrations for infection in MDCK cells. Viral RNA copies in cell lysate were measured by RT-qPCR at 5 h post infection. Relative replication is measured as the percentage of RNA copies of treated samples relative to those of untreated virus. **Figure 1C** is a bar graph showing the antiviral activity of U4, U5, and P16 peptides against A(H1N1) virus as measured by plaque reduction assay (n=3). **Figure 1D** is a bar graph showing the antiviral activity of P16 against A(H1N1) virus as measured by RT-qPCR to quantify the viral RNA copies in supernatants at 18 h post infection (n=3). ** indicates *P*<0.01. *P* value was calculated by the two-tailed Student's *t* test when compared with PBS. **Figure 1E** is a bar graph showing the antiviral viral activity of P16 against A(H3N2) and influenza B (FluB) viruses as measured by a plaque reduction assay (n=4). **Figure 1F** is a bar graph quantifying cytotoxicity of P16 in MDCK cells (n=3). **Figure 1G** is a bar graph showing the percentage hemolysis of turkey red blood cells (TRBC) by P16. Hemolysis (%) was normalized to TRBC treated by Triton X-100. Data are presented as mean ±SD of three independent biological samples.

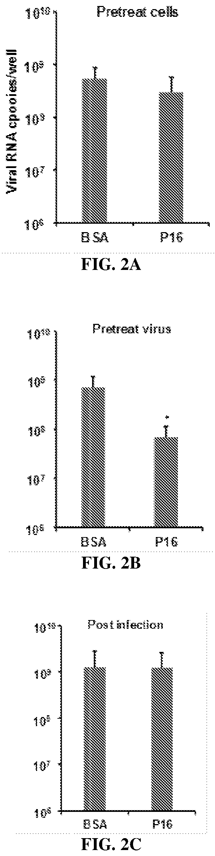

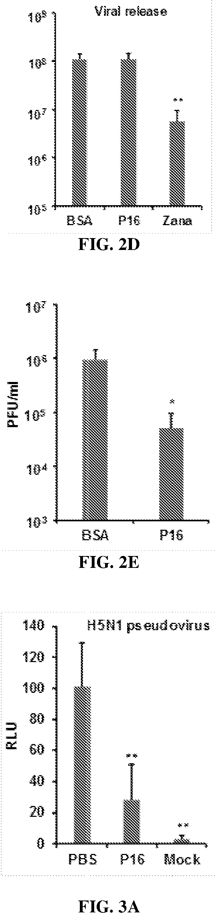

**Figures 2A-2E** show that P16 targets virus to inhibit viral infection at early stage. **Figures 2A-2D** are bar graphs showing quantification of viral RNA copies/well on the y-axis when cells were treated before viral infection (Fig. 2A; n=4), when virus was pretreated by P16 before viral infection (Fig. 2B; n=5), when cells were treated by P16 after viral infection (Fig. 2C), and when cells were treated by P16 after viral infection (Fig. 2D; n=3). In Figures 2A-2C, H1N1 viral RNA copies were measured at 5h post infection in cell lysate (n=3) and in Figure 2D viral RNA copies in cell supernatants were measured at 8 h post infection. **Figure 2E** is a bar graph quantifying plaque forming units (PFU) upon BSA or P16 treatment. P16 showed irreversible antiviral activity (n=3). Virus (1×10⁶ PFU/ml) was pretreated by P16 (500 µg ml⁻¹) and then was diluted to 1,000 folds for plaque assay. Data are presented as mean ±SD of independent biological samples. * indicates *P*<0.05. ** indicates *P*<0.01. *P* values were calculated by the two-tailed Student's *t* test when compared with BSA.

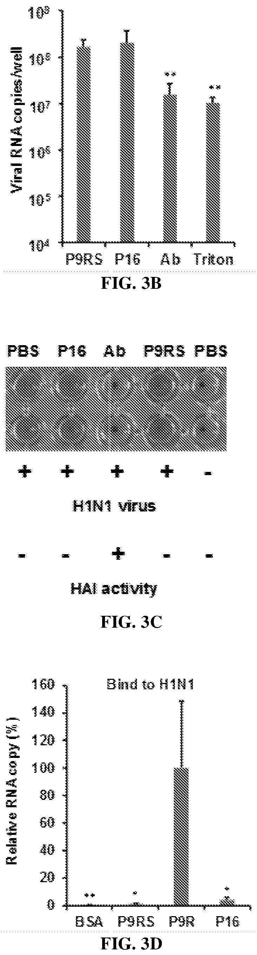

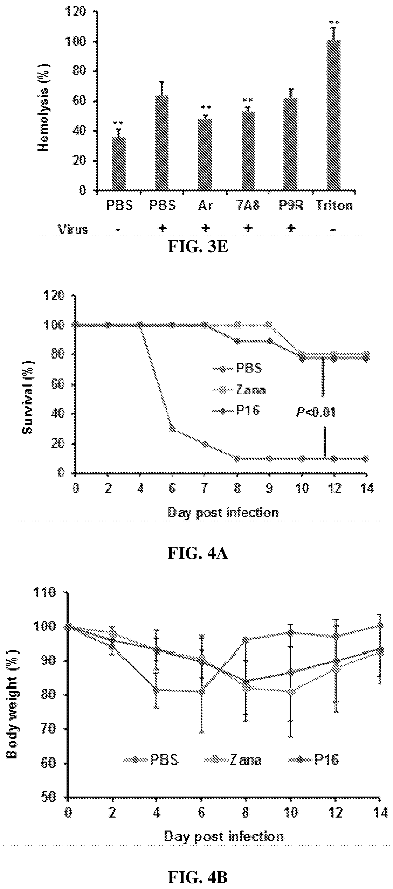

**Figure 3A** is a bar graph quantifying H5N1-pseudovirus cell entry (n=5). H5N1 pseudovirus was pretreated by P16 (50 µg ml⁻¹) for cell infection in 293T cells. Luciferase expression was measured at 24 h post infection. Untreated pseudovirus (PBS) and uninfected cell (mock) were served as controls. ** indicates *P*<0.01. *P* values were calculated when compared with PBS. **Figure 3B** is a bar graph quantifying A(H1N1) viral copies in the indicated conditions (n=3). A(H1N1) virus was pretreated by P16, P9RS (peptide without antiviral activity), neutralizing antibody (Ab) and Triton X-100 for 45 min and then attached to MDCK cells at 4 d for 1h. After washing, attached virus was measured by RT-qPCR. *P* values were calculated when compared with P9RS. **Figure 3C** is an image of the results of the HAI assay. P16 did not show HAI activity (n=3). A(H1N1) virus was pretreated by P16, P9RS, Ab, or PBS. TRBC cells were added to treated virus. Cell precipitates were recorded to show the HAI activity. **Figure 3D** is a bar graph quantifying A(H1N1) viral particle binding activity (n=3). P16, P9R (positive control), P9RS and BSA (negative control) were coated on ELISA plate, which was blocked by BSA. A(H1N1) virus was added to ELISA plate for binding. After washing the unbound virus, viral RNA copies were measured to indicate the bound virus. *P* values were calculated by comparison with P9R. **Figure 3E** is a bar graph quantifying hemolysis induced by A(H1N1) virus at low pH5.0 (n=8). *P* values were calculated by the two-tailed Student's t test when compared with PBS+virus. PBS without virus was the negative control of hemolysis. * indicates *P*<0.05. ** indicates *P*<0.01. Data are presented as mean ±SD of independent biological samples.

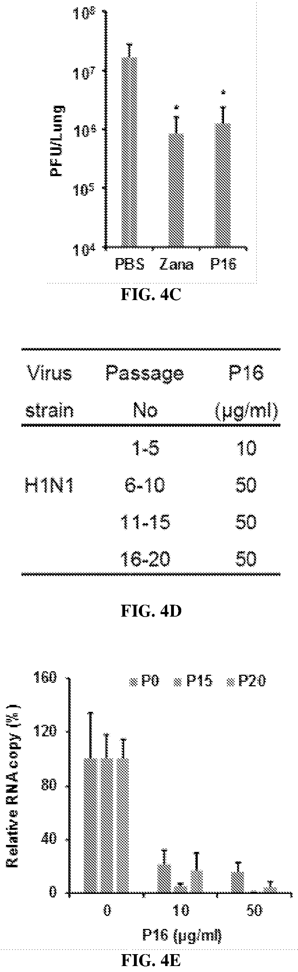

**Figures 4A-4B** are graphs showing the survival (Fig. 4A) and body weight (Fig. 4B) of A(H1N1)-infected mice treated with P16 (50 µg/dose, n=9), zanamivir (Zana, 40 µg/dose, n=5) or PBS (n=9). In Figure 4A, the p-value was calculated by Gehan-Breslow-Wilcoxon test. **Figure 4C** is a bar graph showing A(H1N1) viral loads in mouse lungs treated by P16 (n=5), Zana (n=4) and PBS (n=5). Mice were intranasally inoculated with A(H1N1) virus. At 8 h post infection, indicated drugs were intranasally inoculated to mouse lungs and two more doses were given to mice on the following day. Survivals, body weight and viral loads were measured. * indicates *P*<0.05 when compared with PBS. *P* values were calculated by the two-tailed Student's t test. **Figure 4D** is a table showing the passaging of A(H1N1) virus in the presence of P16. **Figure 4E** is a bar graph showing the antiviral efficiency of P16 against parent H1N1 (P0, n=3), 15-passaged virus (P15, n=3) and 20-passaged virus (P20, n=3). Virus was pretreated with the indicated P16 concentrations for 30 min then infected MDCK cells. After 1h infection, infectious media was removed and fresh media with the indicated concentration of P16 was added to cells for viral culture. Viral RNA copies were measured at 18 h post infection. Relative RNA copy of P16-treated virus was normalized to the RNA copied of virus treated by PBS (0). Data are presented as mean ±SD of independent biological samples.

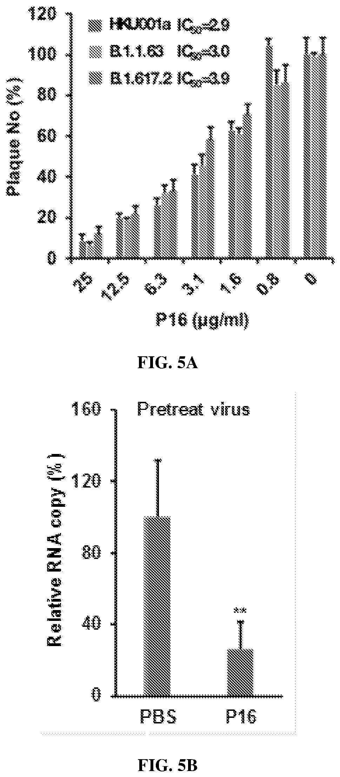

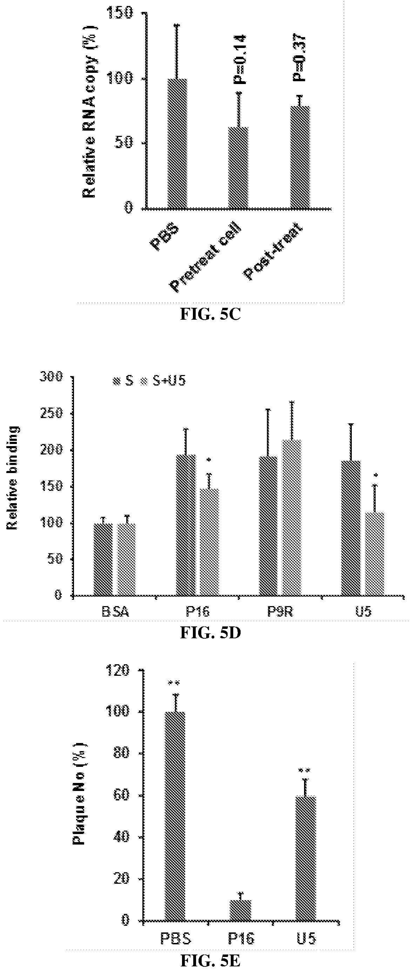

**Figure 5A** is a bar graph quantifying antiviral activity of P16 against SARS-CoV-2 in Vero-E6 cells (n=3). SARS-CoV-2 was premixed with P16 for cell infection. Plaque No (%) was plaque No of P16-treated virus normalized to plaque No of untreated virus. **Figure 5B** is a bar graph quantifying SARS-CoV-2 viral RNA copies in control (PBS) or P16 treatment. SARS-CoV-2 was pretreated by P16 and then infected cells for 1h. Viral RNA copies in cell lysate were measured by RT-qPCR at 8 h post infection (n=5). **Figure 5C** is a bar graph quantifying SARS-CoV-2 viral RNA copies when cell were pre- or post-treated with P16. Cells were pretreated by P16 (Pretreat cell) before viral infection and P16 was added to cells at 1 h post infection (Post-treat). At 8h post infection, viral RNA copies in cell lysate were measured by RT-qPCR (n=3). ** indicates *P*<0.01 when compared with PBS. **Figure 5D** is a bar graph quantifying S protein binding activity of the indicated peptides. P16 bound to S protein and U5 blocked P16 binding to S protein (n=4). S protein of SARS-CoV-2 and S treated by U5 (S+U5) were added to ELISA plate for binding to peptides coated on ELISA plate. * indicates *P*<0.05 when compared with untreated S. **Figure 5E** is a bar graph quantifying antiviral activity of U5 and P16. U5 showed weaker antiviral activity than that of P16 against SARS-CoV-2 (n=4). The antiviral activity was measured by plaque reduction assay. ** indicates *P*<0.01 when compared with P16. *P* values were calculated by the two-tailed Student's t test. Data are presented as mean ±SD of independent biological samples.

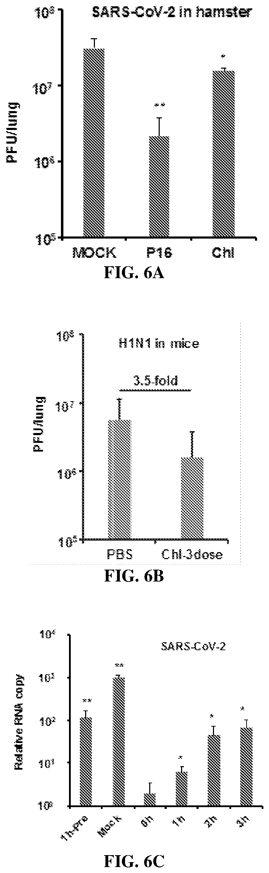

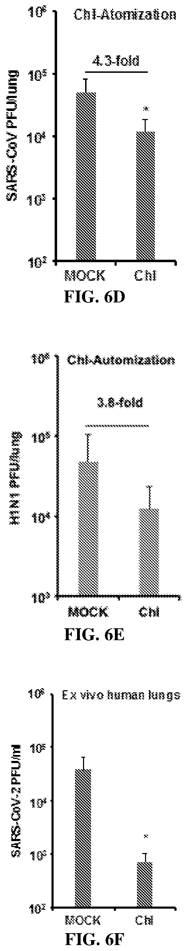

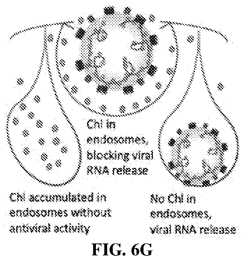

**Figure 6A** is a bar graph showing P16 and chloroquine (Chl, 2 mg kg-1) inhibited SARS-CoV-2 replication in hamster lungs at day 2 post infection (n=4). **Figure 6B** is a bar graph showing chloroquine (2 mg kg-1) inhibited A(H1N1) virus replication in mouse lungs at day 2 post infection (n=8). Drugs were intranasally inoculated to animals at 6 h post infection with two more doses in the following day. **Figure 6C** is a bar graph showing chloroquine more effectively inhibited viral replication during viral infection (n=4). Chloroquine was added to cells at the indicated time points. Viral RNA copies in supernatants were measured at 24h post infection. * indicates P<0.05 and ** indicates P<0.01 when compared with '0h'. **Figures 6D-6E** are bar graphs showing atomization inhalation of chloroquine (50 mg/mouse, one dose) inhibited SARS-CoV (Fig. 6D; n=4) and A(H1N1) virus (Fig. 6E; n=8) replication in mouse lungs at day 1 post infection. **Figure 6F** is a bar graph showing chloroquine (5µg ml-1) significantly inhibited SARS-CoV-2 replication in ex vivo human lung tissues (n=4). Viral RNA copies in supernatants were measured at 60 h post infection. **Figure 6G** is a schematic illustration of functional chloroquine against pH-dependent virus. * indicates P<0.05 and ** indicates P<0.01 when compared with mock. P values were calculated by the two-tailed Student's t test. Data are presented as mean ±SD of independent biological samples.
**Methods of treatment do not form part of the claimed invention and all mention thereof should be considered to be the antiviral agents and compositions thereof for use in such methods.**

### DETAILED DESCRIPTION OF THE INVENTION

The disclosed methods and compositions can be understood more readily by reference to the following detailed description of particular embodiments and the Examples included therein and to the Figures and their previous and following description.

Influenza viruses, coronaviruses, and drug-resistant viruses are long-term threats to public health because of a lack of antiviral agents. Agents with broad-spectrum antiviral activities, and low likelihood of inducing drug resistance are urgently needed to combat such viruses.

As described in the working Examples, a peptic inhibitor termed P16 significantly inhibiting influenza A/B virus by binding to HA to block viral fusion has been discovered. Moreover, P16 antagonizes endosomal acidification to suppress influenza virus and SARS-CoV-2 entry through the endocytic pathway. Importantly, endosomal acidification inhibitor P16 or chloroquine can broadly inhibit A(H1N1) virus, SARS-CoV and SARS-CoV-2 replication in mice and hamsters when administrated through intranasal inoculation or atomization inhalation, contrary to reported treatment failure by systemic route. Chloroquine can significantly inhibit SARS-CoV-2 replication in ex vivo human lung tissues. Thus, it has been discovered that endosomal acidification inhibitors (P16 and chloroquine) can broadly inhibit influenza virus and coronavirus replication in vivo, which supports atomization inhalation of chloroquine for treating coronavirus and influenza patients.

P16 was derived from frog defensin by incorporation of amino acid residues with more positive charges than the parent peptide. P16 can inhibit Influenza A/B virus and SARS-CoV-2. *In vitro* mechanistic studies demonstrated that P16 inhibited the low-pH induced HA conformational changes of A(H1N1), A(H7N7) and FluB viruses. In addition, P16 inhibited endosomal acidification and blocked spike-ACE2-mediated viral entry of SARS-CoV-2 through the endocytic pathway. Importantly, it was discovered that endosomal acidification inhibitors (peptidic P16 and chemical chloroquine) could significantly inhibit A(H1N1) virus, SARS-CoV and SARS-CoV-2 replication in mice and hamsters when administrated through intranasal routes (intranasal inoculation or atomization inhalation), in contrary to previous reports of treatment failure by oral chloroquine in clinical trials. It was also discovered that chloroquine could effectively inhibit viral replication when given at the same route as virus challenge. Moreover, chloroquine significantly inhibited SARS-CoV-2 replication in ex vivo human lung tissues.

Altogether, the results illustrate development of a general strategy for antiviral agent development. By modifying antiviral peptides to include more positive charges, this confers multiple antiviral functions to the peptide such as, blocking viral entry proteins (e.g., blocking influenza HA conformational change) and inhibiting endosomal acidification to prevent viral infection through the endocytic pathway (e.g., blocking influenza virus and SARS-CoV-2 fusion in endolysosomes). It was further demonstrated that endosomal acidification inhibitors (P16 and chloroquine) could effectively inhibit influenza virus and coronavirus replication in vivo when administrated through intranasal inoculation or atomization inhalation. The antiviral activity of atomization inhalation of chloroquine provided the evidence that chloroquine might be re-purposed for treating coronavirus and influenza virus diseases when administrated through atomization inhalation to patients, in which atomization inhalation may achieve more effective drug delivery when compared with the conventional inhalation in animal models.

Additional advantages of the disclosed method and compositions will be set forth in part in the description which follows, and in part will be understood from the description, or can be learned by practice of the disclosed method and compositions. The advantages of the disclosed method and compositions will be realized and attained by means of the elements and combinations particularly pointed out in the appended claims.

It is to be understood that the disclosed method and compositions are not limited to specific synthetic methods, specific analytical techniques, or to particular reagents unless otherwise specified, and, as such, can vary. It is also to be understood that the terminology used herein is for the purpose of describing particular embodiments only

### I. Definitions

"Aerosol" as used herein refers to any preparation of a fine mist of particles, which can be in solution or a suspension, whether or not it is produced using a propellant.

An "emulsion" is a composition containing a mixture of non-miscible components homogenously blended together.

By "contact" or "contacting" is meant to allow or promote a state of immediate proximity or physical association between at least two elements. For example, to contact a virus with a disclosed peptide is to provide physical association between the virus and the peptide.

"Hydrophilic" as used herein refers to substances that have strongly polar groups that readily interact with water.

"Hydrophobic" as used herein refers to substances that lack an affinity for water; tending to repel and not absorb water as well as not dissolve in or mix with water.

"Lipophilic" as used herein refers to compounds having an affinity for lipids.

"Parenteral administration", as used herein, means administration by any method other than through the digestive tract or non-invasive topical or regional routes.

As used herein, the term "subject" means any individual, organism or entity. The subject can be a vertebrate, for example, a mammal (e.g., rat, rabbit, mouse, dog, cat, goat, pig, or horse). Thus, the subject can be a human. The term does not denote a particular age or sex. Thus, adult and newborn subjects, whether male or female, are intended to be covered. The subject may be healthy or suffering from or susceptible to a disease, disorder or condition. A patient refers to a subject afflicted with a disease or disorder. The term "patient" includes human and veterinary subjects.

By "pharmaceutically acceptable" is meant a material that can be administered to a subject without causing undesirable biological effects or interacting in a deleterious manner with any of the other components of the pharmaceutical composition in which it is contained.

As used herein, the term "carrier" or "excipient" refers to an organic or inorganic ingredient, natural or synthetic inactive ingredient in a formulation, with which one or more active ingredients are combined.

"Pharmaceutically acceptable salt", as used herein, refers to derivatives of the compounds defined herein, wherein the parent compound is modified by making acid or base salts thereof.

The term, *"in vitro"* refers to an artificial environment and to processes or reactions that occur within an artificial environment. *In vitro* environments include,
in solution or suspension, and cell cultures. The term *"in vivo"* refers to in or associated with an organism, such as an animal.

"Target cells" describes a cell that is recognized, bound by, and/or infected by a virus. In some forms, a target cell expresses a surface protein that facilitates recognition, binding, and/or entry (infection) by the virus.

As used herein, "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

The term "effective amount," or "therapeutically effective amount" as used herein, refers to an amount of an agent that is sufficient to elicit a desired biological and/or a pharmacologic response. In some forms, an "effective amount" or "therapeutically effective amount" means a quantity sufficient to alleviate or ameliorate one or more symptoms of a disorder, disease, or condition being treated. As will be appreciated by the skilled artisan, the effective amount of an agent, e.g., an isolated peptide, may vary depending on various factors, for example, the desired biological response, the site being targeted, and on the agent being used.

As used herein, the terms "reduce" and "inhibit" mean to decrease an activity, response, condition, disease, or other biological parameter. This can include,
the complete ablation of the activity, response, condition, or disease. It is understood that this is typically in relation to a standard or expected value. The reduction or inhibition may be a 10, 20, 30, 40, 50, 60, 70, 80, 90, 100%, or any amount of reduction in between as compared to native or control levels. In some forms, inhibition or reduction is relative to a state prior to administration of one or more therapeutics. In some forms, inhibition or reduction is relative to a control that is not administered one or more therapeutics.

The term "percent (%) sequence identity" describes the percentage of nucleotides or amino acids in a candidate sequence that are identical with the nucleotides or amino acids in a reference nucleic acid sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

"Identity" can be readily calculated by known methods, including,
those described in Computational Molecular Biology, Lesk, A. M., Ed., Oxford University Press, New York, 1988; Biocomputing: Informatics and Genome Projects, *Smith, D. W.,* Ed., Academic Press, New York, 1993; Computer Analysis of Sequence Data, Part I, Griffin, A. M., and Griffin, H. G., Eds., Humana Press, New Jersey, 1994; Sequence Analysis in Molecular Biology, von Heinje, G., Academic Press, 1987; and Sequence Analysis Primer, Gribskov, M. and Devereux, J., Eds., M Stockton Press, New York, 1991; and Carillo, H., and Lipman, D., SIAM J Applied Math., 48: 1073 (1988). Preferred methods to determine identity are designed to give the largest match between the sequences tested. Methods to determine identity and similarity are codified in publicly available computer programs. The percent identity between two sequences can be determined by using analysis software (*i.e.,* Sequence Analysis Software Package of the Genetics Computer Group, Madison Wis.) that incorporates the Needelman and Wunsch, (J. Mol. Biol., 48: 443-453, 1970) algorithm (*e.g.,* NBLAST, and XBLAST). In some forms, the default parameters can be used to determine the identity for the polynucleotides or polypeptides of the present disclosure.

In some forms, the % sequence identity of a given nucleic acid or amino acid sequence C to, with, or against a given nucleic acid or amino acid sequence D (which can alternatively be phrased as a given sequence C that has or includes a certain % sequence identity to, with, or against a given sequence D) is calculated as follows:
100 times the fraction W/Z,
where W is the number of nucleotides or amino acids scored as identical matches by the sequence alignment program in that program's alignment of C and D, and where Z is the total number of nucleotides or amino acids in D. It will be appreciated that where the length of sequence C is not equal to the length of sequence D, the % sequence identity of C to D will not equal the % sequence identity of D to C.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approximately +/- 10%; in other forms the values may range in value either above or below the stated value in a range of approximately +/- 5%; in other forms the values may range in value either above or below the stated value in a range of approximately +/- 2%; in other forms the values may range in value either above or below the stated value in a range of approximately +/- 1%. The preceding ranges are intended to be made clear by context, and no further limitation is implied.

### II. Compositions

Antiviral agents and compositions containing the antiviral agents are provided. The antiviral agents and compositions contain peptides, such as P16
which have potent antiviral activity. P16 is a peptide with broad antiviral activity, capable of inhibiting viral fusion and endosomal acidification, thereby reducing or preventing viral cell entry through the endocytic pathway.

In some forms, the antiviral agent includes one or more copies of P16 containing the amino acid sequence of RGAHIKGRWKSRCHRF (SEQ ID NO:1).

Typically, the P16-like peptide contains from about 15-25 amino acids and/or an amino acid sequence with at least 75% sequence identity to SEQ ID NO:1 **(which is disclosed but not claimed).**

The antiviral agent and compositions thereof can exhibit a number of antiviral properties. For example, in some forms, upon contact with a virus, the P16
(and the antiviral agent or composition thereof) binds to the virus, reduces or prevents fusion of the virus with target cells, inhibits endosomal acidification of target cells, reduces or prevents cell entry of the virus, reduces or prevents infection of target cells by the virus, reduces or prevents replication of the virus, or combinations thereof. The contacting between the antiviral agent can occur *in vitro* or in a subject *in vivo.*

### A. Antiviral Agents

In some forms, the antiviral agent is or includes an antiviral peptide. In some forms, the antiviral agents and/or compositions include antiviral peptides, small molecule active agents, and combinations thereof.

### 1. Antiviral peptides

In examples not claimed, the antiviral peptide preferably includes a defensin protein, a variant thereof, a portion thereof, or a variant of a portion thereof. For example, the antiviral peptide can preferably be a frog defensin protein such as Urumin (IPLRGAFINGRWDSQCHRFSNGAIACA; SEQ ID NO:2), a variant thereof, a portion thereof, or a variant of a portion thereof. Exemplary peptide portions of Urumin include,
U4 (FINGRWDSQCHRFSNGAIACA; SEQ ID NO:3) and U5 (IPLRGAFINGRWDSQCHRFS; SEQ ID NO:4).

The antiviral peptide includes P16 (RGAHIKGRWKSRCHRF; SEQ ID NO: 1). For example, the disclosed antiviral peptide can consists of the sequence of P16 (see SEQ ID NO:1). P16-like peptides include variants of P16, peptides having a sequence that is a portion of P16, or a variant of a portion thereof. P16-like peptides retain antiviral properties of P16. For example, in some forms, the P16-like peptide is characterized in that the P16-like peptide inhibits endosomal acidification and retains virus binding optionally compared to a control (e.g., P16), as determined by an *in vitro* endosomal acidification and a peptide-virus binding assay (P16-like peptides are disclosed but not claimed).

Suitable antiviral peptides peptide can be of any length or size, as long as they retain functionality (e.g., binding to virus, preventing viral fusion, preventing endosomal acidification). In some forms, the antiviral peptide can have a length of up to 30 residues (e.g., from about 15-25 amino acid residues). For example, the antiviral peptide can have a length of about 15-30 residues, such as about 15-20 residues, about 20-25, or about 25-30 residues. In particular forms, the antiviral peptide has a length of 15, 16, 17, 18, 19, 20, or 21 residues. In some preferred forms, the peptide has a length of 16 residues.

**Disclosed but not claimed are** variants of the disclosed peptides, such as the peptide of SEQ ID NO:1, and modifications thereof retaining the same functional activity. For example, suitable peptides can include one or more point mutations or substitutions (e.g., 1, 2, 3, 4, 5 or more mutations) at any amino acid residue of SEQ ID NO:1. Amino acid substitutions in P16 to obtain P16-like peptides preferably include conservative amino acid substitutions, although non-conservative substitutions can also be used.

Examples of conservative amino acid substitutions include those in which the substitution is within one of the five following groups: 1) small aliphatic, nonpolar or slightly polar residues (Ala, Ser, Thr, Pro, Gly); 2) polar, negatively charged residues and their amides (Asp, Asn, Glu, Gln); polar, positively charged residues (His, Arg, Lys); large aliphatic, nonpolar residues (Met, Leu, Ile, Val, Cys); and large aromatic resides (Phe, Tyr, Trp). Examples of non-conservative amino acid substitutions are those where 1) a hydrophilic residue, e.g., seryl or threonyl, is substituted for (or by) a hydrophobic residue, e.g., leucyl, isoleucyl, phenylalanyl, valyl, or alanyl; 2) a cysteine or proline is substituted for (or by) any other residue; 3) a residue having an electropositive side chain, e.g., lysyl, arginyl, or histidyl, is substituted for (or by) an electronegative residue, e.g., glutamyl or aspartyl; or 4) a residue having a bulky side chain, e.g., phenylalanine, is substituted for (or by) a residue that does not have a side chain, e.g., glycine.

It is understood, however, that substitutions at the recited amino acid positions can be made using any amino acid or amino acid analog. For example, the substitutions at the recited positions can be made with any of the naturally occurring amino acids (e.g., alanine, aspartic acid, asparagine, arginine, cysteine, glycine, glutamic acid, glutamine, histidine, leucine, valine, isoleucine, lysine, methionine, proline, threonine, serine, phenylalanine, tryptophan, or tyrosine).

Alanine scanning of peptides is useful for identifying amino acids that can be modified without reducing binding or other properties of the peptide.

The term "variant" refers to a polypeptide or polynucleotide that differs from a reference polypeptide or polynucleotide, but retains essential properties. A typical variant of a polypeptide differs in amino acid sequence from another, reference polypeptide. Generally, differences are limited so that the sequences of the reference polypeptide and the variant are closely similar overall and, in many regions, identical, but in all cases retain the same functional activity or mechanism of action. A variant and reference polypeptide may differ in amino acid sequence by one or more modifications (*e.g*., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

In preferred forms, the antiviral peptide
has an overall net positive charge. For example, the P16 can have a net positive charge of at least 1, 2, 3, 4, 5 or 6. Preferably, the P16
has a net positive charge of at least 6. In some preferred forms, the P16
   has a net positive charge of about 6.1. Methods of determining the net charge of peptides are known in the art and include, PepCalc by InnovaGen (see https://pepcalc.com/; Lear S., et al., J Comput Aided Mol Des., 30(3):271-7 (2016)). In some forms, the net charge is determined at neutral pH.

**In examples not claimed,** suitable antiviral peptides include peptides having at least 70%, 75%, 80%, 85%, 86%, 87%, 88%, 89%, 90%, 91%, 92%, 93%, 94%, 95%, 96%, 97%, 98%, 99%, sequence identity to SEQ ID NO:1. Thus, a P16-like peptide or peptide derived therefrom has 70%, 75%, 80%, 85%, 86%, 87%, 88%, 89%, 90%, 91%, 92%, 93%, 94%, 95%, 96%, 97%, 98%, 99%, or more sequence identity to SEQ ID NO:1.

It is important however that any modification of the P16 structure ensures that resulting peptide retains inhibition of endosomal acidification and retains virus binding to the same extent as P16. Therefore, useful P16-derived peptides (herein, P16-like peptides) possess the properties of "inhibition of endosomal acidification" and "virus binding". It is within the abilities of one of ordinary skill in the art to vary the amino acids in P16 and test for the required activities (inhibition of endosomal acidification and virus binding) as shown in the examples of this application.

An exemplary "virus-binding assay includes the following steps: Dissolving the peptides (0.1 µg per well) in H₂O and coating onto ELISA plates, then incubating at 4°C overnight. Then, 2% BSA is added to block plates at 4°C overnight. For virus binding to peptides, viruses are diluted in phosphate buffer and then added to ELISA plate for binding to the coated peptides at room temperature for 1h. After washing the unbinding viruses, the binding viruses are lysed by RLT buffer of RNeasy Mini Kit (Qiagen, Cat# 74106) for viral RNA extraction. Viral RNA copies of binding viruses were measured by RT-qPCR.

An exemplary "endosomal acidification assay" can include detecting endosomal acidification with a pH-sensitive dye (pHrodo Red dextran, Invitrogen, Cat^{#}P10361) according to the manufacturer's instructions as previously described but with slight modification (Zhao et al., Nat Commun 9, 2358 (2018)). First, MDCK cells are treated with BSA (25.0 µg ml⁻¹), P16 (25.0 µg ml⁻¹), or other peptides at 4°C for 15 min. Second, MDCK cells are added with 100 µg ml⁻¹ of pH-sensitive dye and DAPI and then incubated at 4 °C for 15 min. Before taking images, cells are further incubated at 37 °C for 15 min and then cells were washed twice with PBS. Finally, PBS is added to cells and images were taken immediately with confocal microscope (for example, Carl Zeiss LSM 700, Germany).

### 2. Peptide modifications

The disclosed peptides may be modified in various ways. In some forms, the modification(s) may render the peptides more stable (e.g., resistant to degradation *in vivo*) or confer other desirable characteristics as will be appreciated by one skilled in the art. Such modifications include, chemical modification, N terminus modification, C terminus modification, peptide bond modification, backbone modifications, residue modification, D-amino acids, or non-natural amino acids or others. In some forms, one or more modifications may be used simultaneously. In preferred forms, the peptides are stabilized against proteolysis. For example, the stability and activity of peptides can be improved by protecting some of the peptide bonds with N-methylation or C-methylation. It is believed that modifications, such as amidation, also enhance the stability of peptides to peptidases.

Modifications to the peptides generally should leave them functional. It is understood that there are numerous amino acid analogs which can be incorporated into the peptides. For example, there are numerous D amino acids or other non-natural amino acids which can be used. The opposite stereoisomers of naturally occurring peptides are disclosed, as well as the stereo isomers of peptide analogs. Amino acid analogs and peptide analogs often have enhanced or desirable properties, such as, more economical production, greater chemical stability, enhanced pharmacological properties (half-life, absorption, potency, efficacy, etc.), altered specificity (e.g., a broad-spectrum of biological activities), reduced antigenicity, and others.

Either or both ends of a given linear peptide can be modified. For example, the peptides can be acetylated and/or amidated.

The peptides may contain naturally occurring α-amino acid residues, non naturally occurring α-amino acid residues, and combinations thereof. The D-enantiomer ("D-α-amino acid") of residues may also be used. Incorporation of artificial amino acids such as beta or gamma amino acids and those containing non-natural side chains, and/or other similar monomers such as hydroxyacids are also contemplated, with the effect that the corresponding component is peptide-like in this respect.

Non-naturally occurring amino acids are not found or have not been found in nature, but they can by synthesized and incorporated into a peptide chain. Non-limiting examples of suitable non-natural amino acids (in L- or D-configuration) are azidoalanine, azidohomoalanine, 2-amino-5-hexynoic acid, norleucine, azidonorleucine, L-α-aminobutyric acid, 3-(1-naphthyl)-alanine, 3-(2- naphthyl)-alanine, p-ethynyl-phenylalanine, m-ethynyl-phenylalanine, p-ethynyl- phenylalanine, p-bromophenylalanine, p-idiophenylalanine, p-azidophenylalanine, and 3-(6- chloroindolyl) alanin.

In some forms, peptide bonds (-CO-NH-) within the peptide may be substituted, for example, by N-methylated bonds (-N(CH3)-CO-), ester bonds (-C(R)H-C-0-0-C(R)-N-), ketomethylen bonds (-CO-CH2-), CC-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the normal side chain, naturally presented on the carbon atom. These modifications can occur at any of the bonds along the peptide chain and even at several (e.g., 2, 3, 4 or more) at the same time.

The peptides can be utilized in a linear form, although it will be appreciated that in cases where cyclization does not severely interfere with peptide characteristics, cyclic forms of the peptides can also be used.

Peptidomimetics may optionally be used to inhibit degradation of the peptides by enzymatic or other degradative processes. The peptidomimetics can be produced by organic synthetic techniques. Non-limiting examples of suitable peptidomimetics include D amino acids of the corresponding L amino acids. D-amino acids can be used to generate more stable peptides, because D amino acids are not recognized by peptidases and such. Systematic substitution of one or more amino acids of a consensus sequence with a D-amino acid of the same type (e.g., D-lysine in place of L-lysine) can be used to generate more stable peptides as long as activity is preserved.

### III. Formulations

The peptides and other active agents disclosed herein described herein can be formulated for enteral, parenteral, or pulmonary administration. In some preferred forms, the peptide and optionally other active agents, is formulated for pulmonary administration.

The disclosed active agents including e.g., P16, or peptides derived therefrom, and/or other antiviral agents such as arbidol, chloroquine and/or camostat can be combined with one or more pharmaceutically acceptable carriers and/or excipients that are considered safe and effective and can be administered to an individual without causing undesirable biological side effects or unwanted interactions. The carrier is all components present in the pharmaceutical formulation other than the active ingredient or ingredients.

The active agents each alone or in any combination can also be formulated for use as a disinfectant, for example, in a hospital environment.

### 1. Pulmonary Formulations

In some forms, the one or more of the antiviral agents or compositions is formulated for pulmonary delivery, such as intranasal administration or oral inhalation.

The respiratory tract is the structure involved in the exchange of gases between the atmosphere and the blood stream. The lungs are branching structures ultimately ending with the alveoli where the exchange of gases occurs. The alveolar surface area is the largest in the respiratory system and is where drug absorption occurs. The alveoli are covered by a thin epithelium without cilia or a mucus blanket and secrete surfactant phospholipids. The respiratory tract encompasses the upper airways, including the oropharynx and larynx, followed by the lower airways, which include the trachea followed by bifurcations into the bronchi and bronchioli. The upper and lower airways are called the conducting airways. The terminal bronchioli then divide into respiratory bronchiole, which then lead to the ultimate respiratory zone, the alveoli, or deep lung. The deep lung, or alveoli, is the primary target of inhaled therapeutic aerosols for systemic drug delivery.

Pulmonary administration of therapeutic compositions including low molecular weight drugs has been observed, for example, beta-androgenic antagonists to treat asthma. Other therapeutic agents that are active in the lungs have been administered systemically and targeted via pulmonary absorption. Nasal delivery is considered to be a promising technique for administration of therapeutics for the following reasons: the nose has a large surface area available for drug absorption due to the coverage of the epithelial surface by numerous microvilli, the sub epithelial layer is highly vascularized, the venous blood from the nose passes directly into the systemic circulation and therefore avoids the loss of drug by first-pass metabolism in the liver, it offers lower doses, more rapid attainment of therapeutic blood levels, quicker onset of pharmacological activity, fewer side effects, high total blood flow per cm3, porous endothelial basement membrane, and it is easily accessible.

Carriers for pulmonary formulations can be divided into those for dry powder formulations and for administration as solutions. Aerosols for the delivery of therapeutic agents to the respiratory tract are known in the art. Aerosols can be produced using standard techniques, such as ultrasonication or high-pressure treatment. For administration via the upper respiratory tract, the formulation can be formulated into a solution, e.g., water or isotonic saline, buffered or un-buffered, or as a suspension, for intranasal administration as drops or as a spray. Preferably, such solutions or suspensions are isotonic relative to nasal secretions and of about the same pH, ranging e.g., from about pH 4.0 to about pH 7.4 or, from pH 6.0 to pH 7.0. Buffers should be physiologically compatible and include, simply by way of example, phosphate buffers. For example, a representative nasal decongestant is described as being buffered to a pH of about 6.2. One skilled in the art can readily determine a suitable saline content and pH for an innocuous aqueous solution for nasal and/or upper respiratory administration.

Preferably, the aqueous solution is water, physiologically acceptable aqueous solutions containing salts and/or buffers, such as phosphate buffered saline (PBS), or any other aqueous solution acceptable for administration to an animal or human. Such solutions are well known to a person skilled in the art and include,
distilled water, de-ionized water, pure or ultrapure water, saline, phosphate-buffered saline (PBS). Other suitable aqueous vehicles include, Ringer's solution and isotonic sodium chloride. Aqueous suspensions can include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

Solvents that are low toxicity organic (i.e. nonaqueous) class 3 residual solvents, such as ethanol, acetone, ethyl acetate, tetrahydofuran, ethyl ether, and propanol can be used for the formulations. The solvent is selected based on its ability to readily aerosolize the formulation. The solvent should not detrimentally react with the P16.

An appropriate solvent should be used that dissolves the compounds or forms a suspension of P16 . The solvent should be sufficiently volatile to enable formation of an aerosol of the solution or suspension. Additional solvents or aerosolizing agents, such as freons, can be added as desired to increase the volatility of the solution or suspension.

In some forms, compositions can contain minor amounts of polymers, surfactants, or other excipients well known to those of the art. In this context, "minor amounts" means no excipients are present that might affect or mediate uptake of P16
in the lungs and that the excipients that are present are present in amount that do not adversely affect uptake of P16 in the lungs.

Dry lipid powders can be directly dispersed in ethanol because of their hydrophobic character. For lipids stored in organic solvents such as chloroform, the desired quantity of solution is placed in a vial, and the chloroform is evaporated under a stream of nitrogen to form a dry thin film on the surface of a glass vial. The film swells easily when reconstituted with ethanol. To fully disperse the lipid molecules in the organic solvent, the suspension is sonicated. Nonaqueous suspensions of lipids can also be prepared in absolute ethanol using a reusable PARI LC Jet+ nebulizer (PARI Respiratory Equipment, Monterey, CA).

Dry powder formulations ("DPFs") with large particle size have improved flowability characteristics, such as less aggregation, easier aerosolization, and potentially less phagocytosis. Dry powder aerosols for inhalation therapy are generally produced with mean diameters primarily in the range of less than 5 microns, although a preferred range is between one and ten microns in aerodynamic diameter. Large "carrier" particles (containing no drug) have been co-delivered with therapeutic aerosols to aid in achieving efficient aerosolization among other possible benefits.

Polymeric particles can be prepared using single and double emulsion solvent evaporation, spray drying, solvent extraction, solvent evaporation, phase separation, simple and complex coacervation, interfacial polymerization, and other methods well known to those of ordinary skill in the art. Particles can be made using methods for making microspheres or microcapsules known in the art. The preferred methods of manufacture are by spray drying and freeze drying, which entails using a solution containing the surfactant, spraying to form droplets of the desired size, and removing the solvent.

The particles can be fabricated with the appropriate material, surface roughness, diameter and tap density for localized delivery to selected regions of the respiratory tract such as the deep lung or upper airways. For example, higher density or larger particles can be used for upper airway delivery. Similarly, a mixture of different sized particles, provided with the same or different EGS can be administered to target different regions of the lung in one administration.

Formulations for pulmonary delivery include unilamellar phospholipid vesicles, liposomes, or lipoprotein particles. Formulations and methods of making such formulations containing nucleic acid are well known to one of ordinary skill in the art. Liposomes are formed from commercially available phospholipids supplied by a variety of vendors including Avanti Polar Lipids. Inc. (Birmingham, Ala.). In some forms, the liposome can include a ligand molecule specific for a receptor on the surface of the target cell to direct the liposome to the target cell.

### 2. Parenteral Formulations

Active agents can be formulated for parenteral administration. For example, parenteral administration can include administration to a patient intravenously, intradermally, intraarterially, intraperitoneally, intracranially, intraarticularly, intraprostatically, intrapleurally, intratracheally, intravitreally, intratumorally, intramuscularly, subcutaneously, subconjunctivally, intravesicularly, intrapericardially, intraumbilically, by injection, and by infusion.

Parenteral formulations can be prepared as aqueous compositions using techniques is known in the art. Typically, such compositions can be prepared as injectable formulations, for example, solutions or suspensions; solid forms suitable for using to prepare solutions or suspensions upon the addition of a reconstitution medium prior to injection; emulsions, such as water-in-oil (w/o) emulsions, oil-in-water (o/w) emulsions, and microemulsions thereof, liposomes, or emulsomes.

The carrier can be a solvent or dispersion medium containing, for example, water, ethanol, one or more polyols (e.g., glycerol, propylene glycol, and liquid polyethylene glycol), oils, such as vegetable oils (e.g., peanut oil, corn oil, sesame oil, etc.), and combinations thereof. The proper fluidity can be maintained, for example, by the use of a coating, such as lecithin, by the maintenance of the required particle size in the case of dispersion and/or by the use of surfactants. In many cases, it will be preferable to include isotonic agents, for example, sugars or sodium chloride.

Solutions and dispersions of the active compounds as the free acid or base or pharmacologically acceptable salts thereof can be prepared in water or another solvent or dispersing medium suitably mixed with one or more pharmaceutically acceptable excipients including, surfactants, dispersants, emulsifiers, pH modifying agents, viscosity modifying agents, and combination thereof.

Suitable surfactants can be anionic, cationic, amphoteric or nonionic surface-active agents. Suitable anionic surfactants include, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer^{®} 401, stearoyl monoisopropanolamide, and polyoxyethylene hydrogenated tallow amide. Examples of amphoteric surfactants include sodium N-dodecyl-.beta.-alanine, sodium N-lauryl-.beta.-iminodipropionate, myristoamphoacetate, lauryl betaine and lauryl sulfobetaine.

The formulation can contain a preservative to prevent the growth of microorganisms. Suitable preservatives include, parabens, chlorobutanol, phenol, sorbic acid, and thimerosal. The formulation can also contain an antioxidant to prevent degradation of the active agent(s).

The formulation is typically buffered to a pH of 3-8 for parenteral administration upon reconstitution. Suitable buffers include, phosphate buffers, acetate buffers, and citrate buffers.

Water-soluble polymers are often used in formulations for parenteral administration. Suitable water-soluble polymers include,
polyvinylpyrrolidone, dextran, carboxymethylcellulose, and polyethylene glycol.

Sterile injectable solutions can be prepared by incorporating P16 in the required amount in the appropriate solvent or dispersion medium with one or more of the excipients listed above, as required, followed by filtered sterilization. Generally, dispersions are prepared by incorporating the various sterilized active ingredients into a sterile vehicle which contains the basic dispersion medium and the required other ingredients from those listed above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The powders can be prepared in such a manner that the particles are porous in nature, which can increase dissolution of the particles. Methods for making porous particles are well known in the art.

### (a) Controlled Release Formulations

The parenteral formulations described herein can be formulated for controlled release including immediate release, delayed release, extended release, pulsatile release, and combinations thereof.

### i. Nano- and microparticles

For parenteral administration, active agents can be incorporated into microparticles, nanoparticles, or combinations thereof that provide controlled release of the active agents. In forms wherein the formulations contains two or more drugs, the drugs can be formulated for the same type of controlled release (e.g., delayed, extended, immediate, or pulsatile) or the drugs can be independently formulated for different types of release (e.g., immediate and delayed, immediate and extended, delayed and extended, delayed and pulsatile, etc.).

For example, active agents can be incorporated into polymeric microparticles, which provide controlled release of the drug(s). Release of the drug(s) is controlled by diffusion of the drug(s) out of the microparticles and/or degradation of the polymeric particles by hydrolysis and/or enzymatic degradation. Suitable polymers include ethylcellulose and other natural or synthetic cellulose derivatives.

Polymers, which are slowly soluble and form a gel in an aqueous environment, such as hydroxypropyl methylcellulose or polyethylene oxide, can also be suitable as materials for drug containing microparticles. Other polymers include,
polyanhydrides, poly(ester anhydrides). polyhydroxy acids, such as polylactide (PLA), polyglycolide (PGA), poly(lactide-co-glycolide) (PLGA), poly-3-hydroxybutyrate (PHB) and copolymers thereof, poly-4-hydroxybutyrate (P4HB) and copolymers thereof, polycaprolactone and copolymers thereof, and combinations thereof.

Alternatively, the drug(s) can be incorporated into microparticles prepared from materials which are insoluble in aqueous solution or slowly soluble in aqueous solution, but are capable of degrading within the GI tract by means including enzymatic degradation, surfactant action of bile acids, and/or mechanical erosion. As used herein, the term "slowly soluble in water" refers to materials that are not dissolved in water within a period of 30 minutes. Preferred examples include fats, fatty substances, waxes, wax-like substances and mixtures thereof. Suitable fats and fatty substances include fatty alcohols (such as lauryl, myristyl stearyl. cetyl or cetostearyl alcohol), fatty acids and derivatives, including fatty acid esters, fatty acid glycerides (mono-, di- and tri-glycerides), and hydrogenated fats. Specific examples include,
hydrogenated vegetable oil, hydrogenated cottonseed oil, hydrogenated castor oil, hydrogenated oils available under the trade name Sterotex^{®}, stearic acid, cocoa butter, and stearyl alcohol. Suitable waxes and wax-like materials include natural or synthetic waxes, hydrocarbons, and normal waxes. Specific examples of waxes include beeswax, glycowax, castor wax, carnauba wax, paraffins and candelilla wax. As used herein, a wax-like material is defined as any material, which is normally solid at room temperature and has a melting point of from about 30 to 300°C.

In some cases, it may be desirable to alter the rate of water penetration into the microparticles. To this end, rate-controlling (wicking) agents can be formulated along with the fats or waxes listed above. Examples of rate-controlling materials include certain starch derivatives (e.g., waxy maltodextrin and drum dried corn starch), cellulose derivatives (e.g., hydroxypropylmethyl-cellulose, hydroxypropylcellulose, methylcellulose, and carboxymethyl-cellulose), alginic acid, lactose and talc. Additionally, a pharmaceutically acceptable surfactant (for example, lecithin) can be added to facilitate the degradation of such microparticles.

Proteins, which are water insoluble, such as zein, can also be used as materials for the formation of drug containing microparticles. Additionally, proteins, polysaccharides and combinations thereof, which are water-soluble, can be formulated with drug into microparticles and subsequently cross-linked to form an insoluble network. For example, cyclodextrins can be complexed with individual drug molecules and subsequently cross-linked.

### ii. Method of making Nano- and Microparticles

Encapsulation or incorporation of drug into carrier materials to produce drug-containing microparticles can be achieved through known pharmaceutical formulation techniques. In the case of formulation in fats, waxes or wax-like materials, the carrier material is typically heated above its melting temperature and the drug is added to form a mixture comprising drug particles suspended in the carrier material, drug dissolved in the carrier material, or a mixture thereof. Microparticles can be subsequently formulated through several methods including, the processes of congealing, extrusion, spray chilling or aqueous dispersion. In a preferred process, wax is heated above its melting temperature, drug is added, and the molten wax-drug mixture is congealed under constant stirring as the mixture cools. Alternatively, the molten wax-drug mixture can be extruded and spheronized to form pellets or beads. These processes are known in the art.

For some carrier materials it may be desirable to use a solvent evaporation technique to produce drug-containing microparticles. In this case drug and carrier material are co-dissolved in a mutual solvent and microparticles can subsequently be produced by several techniques including, forming an emulsion in water or other appropriate media, spray drying or by evaporating off the solvent from the bulk solution and milling the resulting material.

In some forms, drug in a particulate form is homogeneously dispersed in a water-insoluble or slowly water soluble material. To minimize the size of the drug particles within the composition, the drug powder itself can be milled to generate fine particles prior to formulation. The process of jet milling, known in the pharmaceutical art, can be used for this purpose. In some forms drug in a particulate form is homogeneously dispersed in a wax or wax like substance by heating the wax or wax like substance above its melting point and adding the drug particles while stirring the mixture. In this case a pharmaceutically acceptable surfactant can be added to the mixture to facilitate the dispersion of the drug particles.

The particles can also be coated with one or more modified release coatings. Solid esters of fatty acids, which are hydrolyzed by lipases, can be spray coated onto microparticles or drug particles. Zein is an example of a naturally water-insoluble protein. It can be coated onto drug containing microparticles or drug particles by spray coating or by wet granulation techniques. In addition to naturally water-insoluble materials, some substrates of digestive enzymes can be treated with cross-linking procedures, resulting in the formation of non-soluble networks. Many methods of cross-linking proteins, initiated by both chemical and physical means, have been reported. One of the most common methods to obtain cross-linking is the use of chemical cross-linking agents. Examples of chemical cross-linking agents include aldehydes (gluteraldehyde and formaldehyde), epoxy compounds, carbodiimides, and genipin. In addition to these cross-linking agents, oxidized and native sugars have been used to cross-link gelatin. Cross-linking can also be accomplished using enzymatic means; for example, transglutaminase has been approved as a GRAS substance for cross-linking seafood products. Finally, cross-linking can be initiated by physical means such as thermal treatment, UV irradiation and gamma irradiation.

To produce a coating layer of cross-linked protein surrounding drug containing microparticles or drug particles, a water-soluble protein can be spray coated onto the microparticles and subsequently cross-linked by the one of the methods described above. Alternatively, drug-containing microparticles can be microencapsulated within protein by coacervation-phase separation (for example, by the addition of salts) and subsequently cross-linked. Some suitable proteins for this purpose include gelatin, albumin, casein, and gluten.

Polysaccharides can also be cross-linked to form a water-insoluble network. For many polysaccharides, this can be accomplished by reaction with calcium salts or multivalent cations, which cross-link the main polymer chains. Pectin, alginate, dextran, amylose and guar gum are subject to cross-linking in the presence of multivalent cations. Complexes between oppositely charged polysaccharides can also be formed; pectin and chitosan, for example, can be complexed via electrostatic interactions.

### (b) Injectable/Implantable formulations

The active agents described herein can be incorporated into injectable/implantable solid or semi-solid implants, such as polymeric implants. In some forms, one or more active agents is incorporated into a polymer that is a liquid or paste at room temperature, but upon contact with aqueous medium, such as physiological fluids, exhibits an increase in viscosity to form a semi-solid or solid material. Exemplary polymers include,
hydroxyalkanoic acid polyesters derived from the copolymerization of at least one unsaturated hydroxy fatty acid copolymerized with hydroxyalkanoic acids. The polymer can be melted, mixed with the active substance and cast or injection molded into a device. Such melt fabrication require polymers having a melting point that is below the temperature at which the substance to be delivered and polymer degrade or become reactive. The device can also be prepared by solvent casting where the polymer is dissolved in a solvent and the drug dissolved or dispersed in the polymer solution and the solvent is then evaporated. Solvent processes require that the polymer be soluble in organic solvents. Another method is compression molding of a mixed powder of the polymer and the drug or polymer particles loaded with the active agent.

Alternatively, active agents can be incorporated into a polymer matrix and molded, compressed, or extruded into a device that is a solid at room temperature. For example, active agents can be incorporated into a biodegradable polymer, such as polyanhydrides, polyhydroalkanoic acids (PHAs), PLA, PGA, PLGA, polycaprolactone, polyesters, polyamides, polyorthoesters, polyphosphazenes, proteins and polysaccharides such as collagen, hyaluronic acid, albumin and gelatin, and combinations thereof and compressed into solid device, such as disks, or extruded into a device, such as rods.

The release of the peptides and small molecules from the implant can be varied by selection of the polymer, the molecular weight of the polymer, and/or modification of the polymer to increase degradation, such as the formation of pores and/or incorporation of hydrolyzable linkages. Methods for modifying the properties of biodegradable polymers to vary the release profile of the compounds from the implant are well known in the art.

### 3. Enteral Formulations

Suitable oral dosage forms include tablets, capsules, solutions, suspensions, syrups, and lozenges. Tablets can be made using compression or molding techniques well known in the art. Gelatin or non-gelatin capsules can prepared as hard or soft capsule shells, which can encapsulate liquid, solid, and semi-solid fill materials, using techniques well known in the art. In forms where the formulation is for oral administration involving transit through the gastrointestinal tract, the formulation is preferably coated to protect the peptide from gastrointestinal enzymes.

Formulations can be prepared using a pharmaceutically acceptable carrier. As generally used herein "carrier" includes, diluents, preservatives, binders, lubricants, disintegrators, swelling agents, fillers, stabilizers, and combinations thereof.

Carrier also includes all components of the coating composition, which can include plasticizers, pigments, colorants, stabilizing agents, and glidants.

Examples of suitable coating materials include, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name EUDRAGIT^{®} (Roth Pharma, Westerstadt, Germany), zein, shellac, and polysaccharides.

Additionally, the coating material can contain conventional carriers such as plasticizers, pigments, colorants, glidants, stabilization agents, pore formers and surfactants.

"Diluents", also referred to as "fillers," are typically necessary to increase the bulk of a solid dosage form so that a practical size is provided for compression of tablets or formation of beads and granules. Suitable diluents include,
dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar.

"Binders" are used to impart cohesive qualities to a solid dosage formulation, and thus ensure that a tablet or bead or granule remains intact after the formation of the dosage forms. Suitable binder materials include, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone.

"Lubricants" are used to facilitate tablet manufacture. Examples of suitable lubricants include, magnesium stearate, calcium stearate, stearic acid, glycerol behenate, polyethylene glycol, talc, and mineral oil.

"Disintegrants" are used to facilitate dosage form disintegration or "breakup" after administration, and generally include, starch, sodium starch glycolate, sodium carboxymethyl starch, sodium carboxymethylcellulose, hydroxypropyl cellulose, pregelatinized starch, clays, cellulose, alginine, gums or cross linked polymers, such as cross-linked PVP (Polyplasdone^{®} XL from GAF Chemical Corp).

"Stabilizers" are used to inhibit or retard drug decomposition reactions, which include, by way of example, oxidative reactions. Suitable stabilizers include,
antioxidants, butylated hydroxytoluene (BHT); ascorbic acid, its salts and esters; Vitamin E, tocopherol and its salts; sulfites such as sodium metabisulphite; cysteine and its derivatives; citric acid; propyl gallate, and butylated hydroxyanisole (BHA).

### (a) Controlled Release Enteral Formulations

Oral dosage forms, such as capsules, tablets, solutions, and suspensions, can for formulated for controlled release. For example, active agents can be formulated into nanoparticles, microparticles, and combinations thereof, and encapsulated in a soft or hard gelatin or non-gelatin capsule or dispersed in a dispersing medium to form an oral suspension or syrup. The particles can be formed of the drug and a controlled release polymer or matrix. Alternatively, the drug particles can be coated with one or more controlled release coatings prior to incorporation in to the finished dosage form.

In some forms, one or more active agents are dispersed in a matrix material, which gels or emulsifies upon contact with an aqueous medium, such as physiological fluids. In the case of gels, the matrix swells entrapping the active agents, which are released slowly over time by diffusion and/or degradation of the matrix material. Such matrices can be formulated as tablets or as fill materials for hard and soft capsules.

In still some forms, one or more active agents are formulated into a sold oral dosage form, such as a tablet or capsule, and the solid dosage form is coated with one or more controlled release coatings, such as a delayed release coatings or extended release coatings. The coating or coatings can also contain one or more active agents.

### i. Extended release dosage forms

The extended release formulations are generally prepared as diffusion or osmotic systems, which are known in the art. A diffusion system typically consists of two types of devices, a reservoir and a matrix, and is well known and described in the art. The matrix devices are generally prepared by compressing the drug with a slowly dissolving polymer carrier into a tablet form. The three major types of materials used in the preparation of matrix devices are insoluble plastics, hydrophilic polymers, and fatty compounds. Plastic matrices include, methyl acrylate-methyl methacrylate, polyvinyl chloride, and polyethylene. Hydrophilic polymers include,
cellulosic polymers such as methyl and ethyl cellulose, hydroxyalkylcelluloses such as hydroxypropyl-cellulose, hydroxypropylmethylcellulose, sodium carboxymethylcellulose, and Carbopol^{®} 934, polyethylene oxides and mixtures thereof. Fatty compounds include, various waxes such as carnauba wax and glyceryl tristearate and wax-type substances including hydrogenated castor oil or hydrogenated vegetable oil, or mixtures thereof.

In certain preferred forms, the plastic material is a pharmaceutically acceptable acrylic polymer, including acrylic acid and methacrylic acid copolymers, methyl methacrylate, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamine copolymer poly(methyl methacrylate), poly(methacrylic acid)(anhydride), polymethacrylate, polyacrylamide, poly(methacrylic acid anhydride), and glycidyl methacrylate copolymers.

In certain preferred forms, the acrylic polymer is comprised of one or more ammonio methacrylate copolymers. Ammonio methacrylate copolymers are well known in the art, and are described in NF XVII as fully polymerized copolymers of acrylic and methacrylic acid esters with a low content of quaternary ammonium groups.

In some preferred forms, the acrylic polymer is an acrylic resin lacquer such as that which is commercially available from Rohm Pharma under the tradename EUDRAGIT t^{®}. In further preferred forms, the acrylic polymer comprises a mixture of two acrylic resin lacquers commercially available from Rohm Pharma under the tradenames EUDRAGIT^{®} RL30D and EUDRAGIT ^{®} RS30D, respectively. EUDRAGIT^{®} RL30D and EUDRAGIT ^{®} RS30D are copolymers of acrylic and methacrylic esters with a low content of quaternary ammonium groups, the molar ratio of ammonium groups to the remaining neutral (meth)acrylic esters being 1:20 in EUDRAGIT ^{®} RL30D and 1:40 in EUDRAGIT^{®} RS30D. The mean molecular weight is about 150,000. EUDRAGIT ^{®} S-100 and EUDRAGIT ^{®} L-100 are also preferred. The code designations RL (high permeability) and RS (low permeability) refer to the permeability properties of these agents. EUDRAGIT ^{®} RL/RS mixtures are insoluble in water and in digestive fluids. However, multiparticulate systems formed to include the same are swellable and permeable in aqueous solutions and digestive fluids.

The polymers described above such as EUDRAGIT ^{®} RL/RS can be mixed together in any desired ratio in order to ultimately obtain a sustained-release formulation having a desirable dissolution profile. Desirable sustained-release multiparticulate systems can be obtained, for instance, from 100% EUDRAGIT^{®} RL, 50% EUDRAGIT^{®} RL and 50% EUDRAGIT t^{®} RS, and 10% EUDRAGIT^{®} RL and 90% EUDRAGIT^{®} RS. One skilled in the art will recognize that other acrylic polymers can also be used, such as, for example, EUDRAGIT^{®} L.

Alternatively, extended release formulations can be prepared using osmotic systems or by applying a semi-permeable coating to the dosage form. In the latter case, the desired drug release profile can be achieved by combining low permeable and high permeable coating materials in suitable proportion.

The devices with different drug release mechanisms described above can be combined in a final dosage form comprising single or multiple units. Examples of multiple units include, multilayer tablets and capsules containing tablets, beads, or granules An immediate release portion can be added to the extended release system by means of either applying an immediate release layer on top of the extended release core using a coating or compression process or in a multiple unit system such as a capsule containing extended and immediate release beads.

Extended release tablets containing hydrophilic polymers are prepared by techniques commonly known in the art such as direct compression, wet granulation, or dry granulation. Their formulations usually incorporate polymers, diluents, binders, and lubricants as well as the active pharmaceutical ingredient. The usual diluents include inert powdered substances such as starches, powdered cellulose, especially crystalline and microcrystalline cellulose, sugars such as fructose, mannitol and sucrose, grain flours and similar edible powders. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders include substances such as starch, gelatin and sugars such as lactose, fructose, and glucose. Natural and synthetic gums, including acacia, alginates, methylcellulose, and polyvinylpyrrolidone can also be used. Polyethylene glycol, hydrophilic polymers, ethylcellulose and waxes can also serve as binders. A lubricant is necessary in a tablet formulation to prevent the tablet and punches from sticking in the die. The lubricant is chosen from such slippery solids as talc, magnesium and calcium stearate, stearic acid and hydrogenated vegetable oils.

Extended release tablets containing wax materials are generally prepared using methods known in the art such as a direct blend method, a congealing method, and an aqueous dispersion method. In the congealing method, the drug is mixed with a wax material and either spray- congealed or congealed and screened and processed.

### ii. Delayed release dosage forms

Delayed release formulations can be created by coating a solid dosage form with a polymer film, which is insoluble in the acidic environment of the stomach, and soluble in the neutral environment of the small intestine.

The delayed release dosage units can be prepared, for example, by coating a drug or a drug-containing composition with a selected coating material. The drug-containing composition can be, e.g., a tablet for incorporation into a capsule, a tablet for use as an inner core in a "coated core" dosage form, or a plurality of drug-containing beads, particles or granules, for incorporation into either a tablet or capsule. Preferred coating materials include bioerodible, gradually hydrolyzable, gradually water-soluble, and/or enzymatically degradable polymers, and can be conventional "enteric" polymers. Enteric polymers, as will be appreciated by those skilled in the art, become soluble in the higher pH environment of the lower gastrointestinal tract or slowly erode as the dosage form passes through the gastrointestinal tract, while enzymatically degradable polymers are degraded by bacterial enzymes present in the lower gastrointestinal tract, particularly in the colon. Suitable coating materials for effecting delayed release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename Eudragit^{®} (Rohm Pharma; Westerstadt, Germany), including EUDRAGIT^{®} L30D-55 and L100-55 (soluble at pH 5.5 and above), EUDRAGIT^{®} L-100 (soluble at pH 6.0 and above), EUDRAGIT^{®} S (soluble at pH 7.0 and above, as a result of a higher degree of esterification), and EUDRAGITS^{®} NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylose and guar gum; zein and shellac. Combinations of different coating materials can also be used. Multi-layer coatings using different polymers can also be applied.

The preferred coating weights for particular coating materials can be readily determined by those skilled in the art by evaluating individual release profiles for tablets, beads and granules prepared with different quantities of various coating materials. It is the combination of materials, method and form of application that produce the desired release characteristics, which one can determine only from the clinical studies.

The coating composition can include conventional additives, such as plasticizers, pigments, colorants, stabilizing agents, glidants, etc. A plasticizer is normally present to reduce the fragility of the coating, and will generally represent about 10 wt. % to 50 wt. % relative to the dry weight of the polymer. Examples of typical plasticizers include polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil and acetylated monoglycerides. A stabilizing agent is preferably used to stabilize particles in the dispersion. Typical stabilizing agents are nonionic emulsifiers such as sorbitan esters, polysorbates and polyvinylpyrrolidone. Glidants are recommended to reduce sticking effects during film formation and drying, and will generally represent approximately 25 wt. % to 100 wt. % of the polymer weight in the coating solution. One effective glidant is talc. Other glidants such as magnesium stearate and glycerol monostearates can also be used. Pigments such as titanium dioxide can also be used. Small quantities of an anti-foaming agent, such as a silicone (e.g., simethicone), can also be added to the coating composition.

### IV. Methods of manufacture

Methods of preparing the disclosed peptides and compositions thereof are described. In some forms, the peptides can be obtained commercially, such as from a vendor which provides custom peptide synthesis services. In some forms, peptides having a desired sequence can be synthesized or produced recombinantly. Thus, methods of making the peptides using known techniques are provided.

In some forms, the peptides can be produced by recombinant means (e.g., in bacteria, yeast, fungi, insect, vertebrate or mammalian cells) by methods well known to those skilled in the art. Generally this involves creating a DNA sequence that encodes the desired peptide, placing the DNA in an expression cassette under the control of a particular promoter, expressing the peptide in a host, isolating and/or purifying the expressed peptide and, if required, renaturing the peptide.

DNA encoding the peptide(s) can be prepared by any suitable method as described above, including, for example, cloning and restriction of appropriate sequences or direct chemical synthesis. The nucleic acid can be easily ligated into an appropriate vector containing appropriate expression control sequences (e.g. promoter, enhancer, etc.), and, optionally, containing one or more selectable markers (e.g. antibiotic resistance genes). The nucleic acid sequences encoding the peptides can be expressed in a variety of host cells, including, *E. coli,* other bacterial hosts, yeast, fungus, and various higher eukaryotic cells such as insect cells (e.g. SF3), the COS, CHO and HeLa cells lines, and myeloma cell lines. Once expressed, the recombinant peptide(s) can be purified according to standard procedures of the art, including ammonium sulfate precipitation, affinity columns, column chromatography, gel electrophoresis and the like.

In certain forms, the peptides are chemically synthesized by any of a number of fluid or solid phase peptide synthesis techniques known to those of skill in the art. For example, standard FMOC synthesis is described in the literature (e.g., solid phase peptide synthesis, see E. Atherton, RC Sheppard, Oxford University press (1989), or liquid phase synthesis (where peptides are assembled using a mixed strategy by BOC chemistry and fragment condensation). Solid phase synthesis in which the C-terminal amino acid of the sequence is attached to an insoluble support followed by sequential addition of the remaining amino acids in the sequence is a preferred method for the chemical synthesis of the peptides. Techniques for solid phase synthesis are well known to those of skill in the art and are described, for example, by Barany and Merrifield (1963) Solid-Phase Peptide Synthesis; pp. 3-284 in The Peptides: Analysis, Synthesis, Biology. Vol. 2: Special Methods in Peptide Synthesis, Part A.; Merrifield et al. (1963) J. Am. Chem. Soc, 85: 2149-2156, and Stewart et al. (1984) Solid Phase Peptide Synthesis, 2nd ed. Pierce Chem. Co., Rockford, 111.

Such methods include bench scale solid phase synthesis and automated peptide synthesis in any one of the many commercially available peptide synthesizers. Solid phase synthesis is commonly used and various commercial synthesizers are available, for example automated synthesizers by Applied Biosystems Inc., Foster City, CA; Beckman; MultiSyntech, Bochum, Germany etc. Solution phase synthetic methods may also be used, although this can be less convenient. Functional groups for conjugating the peptide to small molecules, label moieties, peptides, or proteins may be introduced into the molecule during chemical synthesis. In addition, small molecules and label moieties/reporter units may be attached during the synthetic process. Preferably, introduction of the functional groups and conjugation to other molecules minimally affects the structure and function of the subject peptide.

Chemical synthesis typically starts from the C-terminus, to which amino acids are sequentially added using either a Rink amide resin (resulting in an -NH₂ group at the C-terminus of the peptide after cleavage from the resin), or a Wang resin (resulting in an - OH group at the C-terminus). Accordingly, peptides having a C-terminal moiety that may be selected from the group consisting of -H, -OH, -COOH, -CONH₂, and -NH₂ are contemplated for use.

Peptides produced using the disclosed methods can be purified using high pressure liquid chromatography (HPLC). Suitable solvents for dissolving the peptides include neat TFA. Typically, the peptides remain soluble at TFA concentrations of 0.5% to 8% and can be loaded onto reverse phase (RP)-HPLC columns for salt exchange. Exemplary salt exchange methods use 3-4 column volumes of acidic buffer to wash away the TFA counter ion due to its stronger acidity coefficient. Buffers suitable for use in washing away the TFA counter ion include 0.1% HCl in H₂O.

Following removal of TFA, peptides can be eluted with a step gradient. Exemplary elution buffers include 30% acetonitrile (MeCN) vs. 0.1% HCl in H₂O. For acetate exchange, peptides can be loaded from the same diluted TFA solution, washed with 3-4 column volumes of 1% acetic acid (AcOH) in H₂O, followed by 2 column volumes of 0.1 M NH₄OAc in H₂O, pH 4.4. In some embodiments, the column is washed again with 3-4 column volumes of 1% AcOH in H₂O.

Analytical HPLC can be carried out to assess the purity and homogeneity of peptides. An exemplary HPLC column for use in analytical HPLC is a PHENOMENEX^{®} JUPITER^{®} column. A step gradient can be used to separate the peptide composition. In some embodiments, the gradient is from 1%-40% MeCN vs 0.05% TFA in H₂O. The change in gradient can be achieved over 20 minutes using a flow rate of 1 ml/min. Peptides can be detected using UV detection at 215 nm.

Where the peptides or compositions thereof are required to be sterilized or otherwise processed for the removal of undesirable contaminants and/or microorganisms, filtration can be used. Filtration can be achieved using any system or procedures known in the art. In some forms, filtration removes contaminants or prevents the growth or presence of microorganisms. Exemplary microorganisms and contaminants that can be removed include bacteria, cells, protozoa, viruses, fungi, and combinations thereof. In some forms, the step of filtration is carried out to remove aggregated or oligomerized peptides. For example, solutions of the peptides can be filtered to remove oligomers on the basis of size.

### IV. Methods of use

**(Methods of treatment do not form part of the claimed invention. Any reference to methods of treatment should be interpreted as the antiviral agents and compositions as claimed for use in such methods.**

Disclosed herein are various methods related to the disclosed peptides and compositions and their use. The peptides and compositions thereof can be used in therapeutic and/or prophylactic applications. For example, methods of treating a viral infection are provided.

In some forms, a method of treating a viral infection includes administering to the subject an effective amount of any of the disclosed antiviral agents or any of the disclosed antiviral compositions. For example, a subject can be administered an effective amount of a composition containing antiviral agents and a pharmaceutically acceptable carrier. In some preferred forms, the composition is in a form suitable for intranasal or pulmonary delivery. In some forms, the composition is an injectable formulation. In some forms, the composition is administered parenterally or orally. In some forms, the composition is administered intranasally, or by pulmonary administration.

In some forms, the subject to be treated is suffering from an infection from a respiratory virus, and more preferably, a pH-dependent respiratory virus. Respiratory viruses are the most frequent causative agents of disease in humans, with significant impact on morbidity and mortality worldwide, mainly in children. Approximately one-fifth of all childhood deaths worldwide are related to acute respiratory infections (ARIs), particularly in impoverished populations of tropical regions, where ARI case-to-fatality ratios can be remarkably higher than in temperate regions of the world. Eight human respiratory viruses circulate commonly in all age groups and are recognized as adapted to efficient person-to-person transmission; the include HRSV (human respiratory syncytial virus), HPIV (human parainfluenza Virus), HRV (human rhinovirus), ADV (adenovirus), HCoV (human coronavirus) (HCoV-NL63, HCoV-HKU1), SARS-CoV, HMP (human metapneumovirus) HPIV (human parainfluenza virus ) and HBoV (human bocavirus). HRSV internalization is considered to be pH-independent and may happen either in plasma or in endosomal membranes. Thus, infections caused by any of the foregoing respiratory viruses can be treated in accordance with the disclosed methods.

Thus, methods of treating a respiratory viral infection are described. In some forms, the infection is caused by a pH-dependent virus that requires endosomal acidification for virus-host membrane fusion (e.g., fusion that results in escape of the viral genome from the endosome into the cytoplasm).

In some forms, the disclosed compositions and methods are used to treat an infection caused by a virus selected from, zika virus, enterovirus-A7, ebola virus, influenza virus (e.g., influenza A virus, influenza B virus), HRSV, HPIV, HRV, ADV, HCoV, SARS-CoV-2, SARS-CoV, MERS-CoV, and the non-enveloped rhinovirus. Exemplary influenza viruses include, A(H7N9), influenza A(H7N7), influenza A(H5N1), influenza A(H1N1) (e.g., the A(H1N1)pdm09 virus) and influenza A(H3N2).

The subject can be treated with the disclosed peptides and/or other active agents by administering an effective amount of the peptide and/or other active agents to the subject, enterally, by pulmonary or nasal administration, or parenterally (intravenously, intradermally, intraarterially, intraperitoneally, intracranially, intraarticularly, intraprostatically, intrapleurally, intratracheally, intravitreally, intratumorally, intramuscularly, subcutaneously, subconjunctivally, intravesicularly, intrapericardially, intraumbilically, by injection, and by infusion.

Preferably, the subject is a human.

### Effective amounts

Typically, the methods involve administering or applying an effective amount of the antiviral agents or compositions thereof. For example, in some forms, the compositions are administered to a subject in an effective amount for treatment and/or prevention of a disease, disorder or condition caused by a virus (e.g., COVID-19).

The effective amount can be a dosage sufficient to treat, inhibit, or alleviate one or more symptoms of a disorder, disorder or condition being treated or to otherwise provide a desired pharmacologic and/or physiologic effect. The effective amount of the peptide compositions will vary from subject to subject, and can depend on the species, age, weight and general condition of the subject, the severity of the disorder being treated, and its mode of administration. Thus, it is not possible to specify an exact amount for every therapeutic composition. However, an appropriate amount can be determined by one of ordinary skill in the art using only routine experimentation given the teachings herein. For example, effective dosages and schedules for administering the therapeutics may be determined empirically, and making such determinations is within the skill in the art. The dosage ranges for the administration of the compositions are those large enough to effect one or more desired responses.

In some forms, the antiviral agent or composition is administered in an effective amount to reduce one or more symptoms of disease, disorder, or illness associated with a virus or viral infection. Suitable symptoms include fever, congestion in the nasal sinuses and/or lungs, runny or stuffy nose, cough, sneezing, sore throat, body aches, fatigue, shortness of breath, chest tightness, wheezing when exhaling, chills, muscle aches, headache, diarrhea, tiredness, nausea, vomiting, and combinations thereof.

In some forms, the antiviral agent or composition is administered in an effective amount to reduce or prevent viral replication in a subject. In a particular form, the antiviral agent or composition is administered in an effective amount to reduce or prevent spike-ACE2-mediated cell entry of a virus (e.g., SARS-CoV-2). In a particular form, the antiviral agent or composition is administered in an effective amount to inhibit low-pH induced conformational changes in HA present on influenza.

### Combination Therapy

In some forms, the disclosed antiviral agent or composition is administered in combination with one or more additional active agents. The combination therapies can include administration of the active agents together in the same admixture, or in separate admixtures. Therefore, in some forms, the pharmaceutical composition includes two, three, or more active agents. Such formulations typically include an effective amount of antiviral peptide or a pharmaceutically acceptable salt thereof. In some forms, the second active agent is an antiviral (i.e., a second antiviral), a fever reducer, an anti-inflammatory agent, an analgesic, or a combination thereof.

Thus, provided are methods for treating a subject infected with a virus, by administering the subjected a formulation containing an effective amount of the disclosed antiviral peptides in combination with one or more additional active agents, e.g., one or more of arbidol, chloroquine or camostat, to ameliorate one or more symptoms associated with the viral infection.

Any suitable antiviral agent can be used in the combination therapy.

Typically, the antiviral agents are used in a combination that simultaneously blocks two entry pathways of a virus, e.g., a coronavirus such as SARS-CoV-2. For example, in some forms, the combination therapy includes administration of multiple active agents which in combination accomplish at least two or all of: a reduction in endosomal acidification and/or elevation in endosomal pH, a reduction in virus-host cell fusion (e.g., mediated by spike and ACE2), and a reduction in viral entry.

In some forms, the combination therapy includes P16, arbidol, chloroquine, comastat, or combinations thereof.

In some forms, the combination of two or more active agents achieves a result greater than when the individual agents are administered alone or in isolation. For example, in some forms, the result achieved by the combination is partially or completely additive of the results achieved by the individual components alone. In the most some preferred forms, the result achieved by the combination is more than additive of the results achieved by the individual components alone.

In some forms, the effective amount of one or both agents used in combination is lower than the effective amount of each agent when administered separately. In some forms, the amount of one or both agents when used in the combination therapy is sub-therapeutic when used alone.

The combinations can be administered either concomitantly (*e.g.*, as an admixture), separately but simultaneously (*e.g*., via separate intravenous lines into the same subject; one agent is given orally while the other agent is given by infusion or injection, *etc.*,)*,* or sequentially (*e.g.,* one agent is given first followed by the second).

Thus, a treatment regimen of a combination therapy can include one or multiple administrations of each active agent. In certain forms, the two or more agents are administered simultaneously in the same or different pharmaceutical compositions.

In some forms, two or more active agents are administered sequentially, typically, in two or more different pharmaceutical compositions. The different active agents be administered hours or days apart. The additive or more than additive result may be evident after one day, two days, three days, four days, five days, six days, one week, or more than one week following administration.

Dosage regimens or cycles of the agents can be completely or partially overlapping, or can be sequential. In some forms, all such administration(s) of one agent occurs before or after administration of the second and/or third agent. Alternatively, administration of one or more doses of the one or more agents can be temporally staggered.

An effective amount of each of the agents can be administered as a single unit dosage (*e.g.,* as dosage unit), or sub-therapeutic doses that are administered over a finite time interval. Such unit doses can be administered on a daily basis for a finite time period, such as up to 3 days, or up to 5 days, or up to 7 days, or up to 10 days, or up to 15 days or up to 20 days or up to 25 days, are all specifically contemplated.

The present invention will be further understood by reference to the following non-limiting examples.

### EXAMPLES

**The invention is defined in the appended claims. Any of the following examples that do-not fall within the scope of the claims are provided for illustrative or comparative purposes only.**

### Example 1: The antiviral peptide, P16, is a fusion and endosomal acidification inhibitor of influenza virus and SARS-CoV-2.

### Material and Methods

### Cell and virus culture

Madin Darby canine kidney (MDCK, CCL-34), Vero-E6 (CRL-1586) and 293T cells (CRL-3216) obtained from ATCC (Manassas, VA, USA) were cultured in Dulbecco minimal essential medium (DMEM) or MEM supplemented with 10% fetal bovine serum (FBS), 100 IU ml⁻¹ penicillin and 100 µg ml⁻¹ streptomycin. The virus strains used in this study included A/Hong Kong/415742M/2009(H1N1), A/Hong Kong/4801/2014(H3N2), A/Netherlands/219/2003(H7N7), A/Anhui/1/2013(H7N9), influenza B/TW/70555/05(FluB) and SARS-CoV-2 (16, 34).

### Plaque reduction assay

Peptides were synthesized by ChinaPeptide. Antiviral activity of peptides was measured using a plaque reduction assay. Briefly, peptides or bovine serum albumin (BSA, 0.2-50.0 µg ml⁻¹) were premixed with 50 PFU of virus in PBS at room temperature. After 45-60 min of incubation at room temperature, peptide-virus mixture was transferred to MDCK or Vero-E6 cells, correspondingly. At 1 h post infection, infectious media were removed and 1% low melting agar was added to cells. Cells were fixed using 4% formalin at 2-3 day post infection. Crystal violet (0.1%) was added for staining, and the number of plaques was counted.

### Viral RNA extraction and RT-qPCR

Viral RNA was extracted by Viral RNA Mini Kit (QIAGEN, Cat^{#} 52906, USA) according to the manufacturer's instructions. Extracted RNA was reverse transcribed to cDNA using PrimeScript II 1^{st} Strand cDNA synthesis Kit (Takara, Cat^{#} 6210A) with GeneAmp^{®} PCR system 9700 (Applied Biosystems, USA). The cDNA was then amplified using specific primers (Table 1) for detecting A(H1N1) virus using LightCycle^{®} 480 SYBR Green I Master (Roach, USA). For quantitation, 10-fold serial dilutions of standard plasmid equivalent to 10¹ to 10⁶ copies per reaction were prepared to generate the calibration curve. Real-time qPCR experiments were performed using LightCycler^{®} 96 system (Roche, USA).

**Table 1. Primers for RT-qPCR**

| Gene | Primer | Oligonucleotide sequence (5' to3') |
|---|---|---|
| SARS-CoV-2 | S-F | CCTACTAAATTAAATCATCTCTGCTTTACT (SEQ ID NO:8) |
| | S-R | CAAMGCTATAACGCAGCCTGTA (SEQ ID NO:9) |
| H1N1 | M-F | CTTCTAACCGAGGTCGAAACG (SEQ ID NO:10) |
| | M-R | GGC ATTTTGGACAAAKCGTCT A (SEQ ID NO:11) |
| H7N9 | M-F | CTTCTAACCGAGGTCGAAACG (SEQ ID NO:10) |
| | M-R | GGC ATTTTGGACAAAKCGTCT A (SEQ ID NO:11) |

### Antiviral multicycle growth assay

Influenza viruses were pretreated by peptide and then infected MDCK cells (0.005 MOI). After 1h infection, infectious media were removed and fresh media with supplemental peptides were added to infected cells for virus culture. At 18 h post infection, the supernatants of infected cells were collected for RT-qPCR assay to determine the viral titers in cell supernatants.

### Cytotoxicity assay

Cytotoxicity of peptides was determined by the detection of 50% cytotoxic concentration (CC₅₀) using a tetrazolium-based colorimetric MTT assay. Briefly, MDCK cells were seeded in 96-well cell culture plates at an initial density of 2 × 10⁴ cells per well in DMEM supplemented with 10% FBS and incubated overnight. Cell culture media were removed and then DMEM supplemented with various concentrations of peptides and 1% FBS were added to each well. After 24 h incubation at 37 °C, MTT solution (5 mg ml⁻¹, 10 µl per well) was added to each well for incubation at 37 °C for 4 h. Then, 100 µl of 10% SDS in 0.01M HCl was added to each well. After further incubation at room temperature with shaking overnight, the plates were read at OD₅₇₀ using VictorTM X3 Multilabel Reader (PerkinElmer, USA). Cell culture wells without peptides were used as the experiment control and medium only served as a blank control.

### Hemolysis and hemolysis inhibition assay

Serially diluted peptide P16 in PBS was incubated with turkey red blood cells for 1 h at 37°C. PBS was used as a 0% lysis control and 0.1% Triton X-100 as 100% lysis control. Plates were centrifuged at 350 g for 3 min to pellet non-lysed red blood cells. Supernatants used to measure hemoglobin release were detected by absorbance at 450 nm. For hemolysis inhibition assay, P16 (200 µg ml⁻¹), P9R (200 µg ml⁻¹) or Arbidol (100 µg ml⁻¹) were mixed with or without same volume of H1N1 virus (HA titer >128) for 1 h, and then 60 µl of 2% turkey red blood cells was added for 15 min. PBS and Triton X-100 (0.1%) were included as the negative and positive control of hemolysis. The precipitated erythrocytes were incubated with sodium citrate solution (pH of 4.9) for 25 min. The hemoglobin release in supernatants was detected at 450 nm.

### Transmission electron microscopy assay

To determine the effect of P16 on viral particles, A(H1N1) virus was pretreated by 200 µg ml⁻¹ of P16, PBS or Triton X-100 (0.15%) for 1h. The virus was fixed by formalin for overnight and then applied to continuous carbon grids. The grids were transferred into 4% uranyl acetate and incubated for 1 min. After removing the solution, the grids were air-dried at room temperature. For each sample, two-three biological samples were used for taking TEM images by FEI Tecnal G2-20 TEM.

### H5N1 pseudovirus assay

H5N1 pseudotype virus (21) bearing H5N1 HA and NA was pretreated with PBS or P16 in PBS and then incubated at RT for 1h. MDCK cells were infected with the treated pseudotype virus for 1h. MDCK cells without pseudotype virus infection were served as the baseline control of luciferase protein. After 18h cell culture, cell lysates were collected and the luciferase protein was measured by Luciferase assay system (Promega) in a Victor X3 Multilabel reader (PerkinElmer). The luminescence reading was normalized to 1mg protein.

### Trypsin susceptibility assay

A(H1N1) HA was preincubated with peptides or FA617 (21) for 30 min at room temperature. The pH of each reaction was lowered to pH5.0 using 0.25 M citrate (pH4.2). Other reactions were retained at pH7.4. The reaction solutions were incubated at 37°C for 15 min. After incubation, the low pH reaction solutions were neutralized by addition of 0.25M Tris HCl (pH9.2). Trypsin was added to samples for digestion at 37°C for 15 min. After incubation with trypsin, the reaction solutions were quenched by addition of non-reduced SDS buffer and boiled for 3 min. Samples were analyzed by 12% SDS-PAGE gel.

### Virus induced cell fusion assay

MDCK cells were transfected with pGFP. Eight hour later, cells were infected with 1MOI of FluB virus. At 18h post infection, cells were treated by PBS or P16 for 1h and then cells were treated by pH5.0 or pH 7.4 for 10 min. After removing the pH buffer, cells were cultured at 37 °C for 4 h with full media. Fusion pictures were taken at 4h after pH treatment.

### HA mediated cell fusion assay

The 293T cells were co-transfected with pGFP and pH7N7-HA. At 24h post transfection, cells were treated by PBS or P16 for 1h and then treated by pH5.0 or pH 7.4 for 10 min. After removing the pH buffer, cells were cultured at 37 °C for 4 h with full media. Fusion pictures were taken at 4 h after pH treatment.

### Spike-ACE2 mediated cell fusion assay

The pSpike of SARS-CoV-2, pACE2-human, or pGFP were transfected to 293T cells for protein expression. After 24 hours, to trigger the spike-ACE2 mediated cell fusion, 293T-Spike-GFP cell were co-cultured with 293T-ACE2 with or without the supplementation of peptide or drug. The 293T-GFP cells were co-cultured with 293T-ACE2 cells as the no-fusion negative control. After 8 h of co-culture, five fields were randomly selected in each well to take the cell fusion pictures by fluorescence microscopes.

### Peptide binding assay

Peptides (1.0-3.0 µg per well) dissolved in H₂O were coated onto ELISA plates and incubated at 4°C overnight. Then, 2% BSA was used to block plates at 4°C overnight. For virus or spike protein binding to peptides, viruses or spike protein were diluted in PBS and then were added to ELISA plate for binding to the coated peptides at room temperature for 1h. After washing the unbound viruses or spike protein, the bound viruses were lysed by RLT buffer of RNeasy Mini Kit (Qiagen, Cat# 74106) for viral RNA extraction. Viral RNA copies of binding viruses were measured by RT-qPCR. The bound spike protein detected by anti-His-HRP by reading OD₄₅₀.

### Endosomal acidification assay

Endosomal acidification was detected with a pH-sensitive dye (pHrodo Red dextran, Invitrogen, Cat^{#}P10361) according to the manufacturer's instructions as previously described but with slight modification(3). First, MDCK cells were treated with BSA (25.0 µg ml⁻¹), P16 (25.0 µg ml⁻¹) and bafilomycin A1 (50 nM) at 4 °C for 15 min. Second, MDCK cells were added with 100 µg ml⁻¹ of pH-sensitive dye and DAPI and then incubated at 4 °C for 15 min. Before taking images, cells were further incubated at 37 °C for 15 min and then cells were washed twice with PBS. Finally, PBS was added to cells and images were taken immediately with confocal microscope (Carl Zeiss LSM 700, Germany).

### Rearing of virus resistant mutant

A(H1N1) virus was pretreated by P16 and then infected MDCK cells. After 1 h infection, MEM media with the supplement P16 were added to cells for virus culture. At 24 h post infection, viruses were collected for next passages. After 20 passages, passaged viruses (P20 and P15) and virus without passage (P0) were pretreated by P16 or PBS with the indicated concentration of P16 for MDCK infection. At 18 h post infection, viral loads in cell supernatants were measured by RT-qPCR to identify the sensitivity of passaged viruses to P16.

### Hemagglutination inhibition assay

HA titers of H1N1 and FluB viruses were tested by TRBC. Viruses (8HA titer) were premixed with peptides or PBS for 1h and then equal volume of TRBC was added to virus for incubation at room temperature for 30 min. The precipitate of TRBC were recorded for calculating the HAI activity. TRBC with untreated virus and neutralization antibody from serum served as negative and positive controls of hemagglutination inhibition.

### Antiviral analysis in mice

BALB/c female mice, 10-12 weeks old, were kept in biosafety level 2 laboratory (housing temperature between 22-25°C with dark/light cycle) and given access to standard pellet feed and water *ad libitum.* All experimental protocols followed the standard operating procedures of the approved biosafety level 2 animal facilities and were approved by the Committee on the Use of Live Animals in Teaching and Research of the University of Hong Kong (35). The mouse adapted A(H1N1) virus was used for antiviral assay in mice. To evaluate the therapeutic effect, mice were challenged with 3 LD₅₀ of A(H1N1) virus and then intranasally inoculated with PBS, P16 or zanamivir at 8 h after the viral inoculation. Two more doses were given to challenged mice on the following day. Survival and general conditions were monitored for 14 days or until death. Viral loads in mouse lungs were measured at day 2 post infection.

### Human Ex Vivo Lung Tissues

Human lung tissues for ex vivo studies were obtained from patients undergoing surgical operations at Queen Mary Hospital, Hong Kong. All donors gave written consent as approved by the Institutional Review Board of the University of Hong Kong/Hospital Authority Hong Kong West Cluster (UW13-364). The freshly obtained lung tissues were processed into small pieces and then were infected with SARS-CoV-2 (2 × 10⁴ PFU) in 500 µl advanced Dulbecco's Modified Eagle's Medium (DMEM)/F12 medium (Gibco, Thermo Fisher Scientific) supplemented with 100 U/mL penicillin, 100 µg/mL streptomycin and chloroquine (5 µg/ml). After 18 hours, the inoculum was removed and the specimens were washed one time with PBS. The infected human lung tissues were then cultured in 0.5 ml of advanced DMEM/F12 medium with chloroquine (5 µg/ml). Supernatants were collected at 60 hours post inoculation for plaque assays.

### Results

### Basic peptide P16 inhibited influenza A and B virus

Previous studies of mouse beta defensin (30 amino acid peptide) indicated that putative virus-binding peptides without antiviral activity through virus binding could be modified to have more positive charges to enhance the antiviral activity by inhibiting endosomal acidification (16). Here, it was determined whether a virus-binding antiviral peptide could be modified with more positive charges to acquire dual-functional activities against viruses due to the inhibition by direct binding and the enhanced inhibition on endosomal acidification. It was observed that the U4 and U5 peptides, derived from a frog defensin, Urumin, which could bind to HA stem of group 1 influenza A virus (13), showed more potent antiviral activity than that of Urumin against A(H1N1) virus (Fig. 1A-1B). Based on U4 and U5, a short 16 amino acid peptide (P16) with positive charge (+6.1) was identified, which significantly inhibited A(H1N1) virus (IC₅₀=3.9 µg ml⁻¹, Fig. 1C), which is lower than the IC₅₀ of U4 (6.6 µg ml⁻¹) and U5 (12.9 µg ml⁻¹). The potent anti-H1N1 activity of P16 was further confirmed by the inhibition on viral multicycle growth, which showed that P16 inhibited viral replication by 41-fold (Fig. 1D). P16 could also inhibited A(H3N2) (IC₅₀=1.6 µg ml⁻¹) and FluB (IC₅₀=7.1 µg ml⁻¹) viruses (Fig. 1E). Cytotoxicity analysis showed no significant cytotoxicity of P16 on MDCK cells treated by 1 mg ml⁻¹ of P16 (TC₅₀> 1mg ml⁻¹, Fig. 1F) and no significant hemolysis was observed when Turkey red blood cells (RBC) were treated by P16 (Fig. 1G). These results showed that the short basic peptide, P16, potently inhibits influenza A (group 1 and group 2) and B viruses without obvious cytotoxic effect on host cells.

### P16 inhibited HA fusion and endosomal activation

To investigate the antiviral mechanism of P16 against influenza virus, cells were treated with P16 before A(H1N1) virus infection, but no antiviral activity was detected (Fig. 2A). When virus was pretreated by P16 before viral infection, P16 significantly inhibited viral replication (Fig. 2B). When infected cells were treated by P16 after viral infection, P16 did not inhibit viral replication in cells (Fig. 2C) and viral release in supernatants (Fig. 2D). These results indicated that the antiviral activity of P16 relies mainly on targeting virus before viral entry. To further confirm this result, A(H1N1) virus (1×10⁶ PFU ml⁻¹) was treated with P16 (500 µg ml⁻¹) and the P16-treated virus was then diluted by 10, 000 fold for plaque assay. After dilution, P16 (at a concentration of 0.05 µg ml⁻¹, which is less than the IC₅₀ of 3.9 µg ml⁻¹) still significantly reduced the plaque number (Fig. 2E), which further confirmed that the antiviral activity of P16 mainly relied on targeting virus and that the binding of P16 to the virus was irreversible. Next, it was investigated whether P16 disrupted the virus. A(H1N1) virus was pretreated by P16 (200 µg ml-1), P9RS or Triton X-100 (positive control of viral disrupting). Virus was negatively stained for TEM analysis. Intact A(H1N1) virus treated by P16 could be detected by transmission electron microscopy (TEM). However, intact viral particles were not detected when virus was treated by Triton X-100. These results indicated that the antiviral activity of P16 mainly relied on direct targeting of virus which interfered with viral infection at early stage without disrupting the viral particles.

Next, A(HSN1) pseudovirus, which only expressed HA and NA proteins was first used to test whether P16 could affect the entry of pseudovirus, was used. As shown in Fig. 3A, P16 significantly inhibited the entry of A(H5N1) pseudovirus, indicating that P16 likely interfered with the early step of viral infection by targeting HA. P16 did not reduce viral attachment (Fig. 3B), indicating that P16 did not disrupt viral particles because the viral RNA copies of attached virus were significantly reduced on the cell surface (Fig. 3B) when virus was treated and disrupted by Triton X-100. It was confirmed that P16 did not have hemagglutination inhibition (HAI) activity against A(H1N1) and FluB viruses when compared with a neutralization antibody (Fig. 3C). Furthermore, unlike P9R which bound to viral surface HA and captures viral particles (16), P16 could not capture viral particles as shown in capture EIA assay (Fig. 3D), indicating that P16 was less likely to bind to the head region of HA.

Considering the broad-spectrum antiviral activity of P16 against group 1 and 2 influenza A virus and FluB viruses, it was hypothesized that P16 binds to the HA stem region so as to interfere with HA conformational change. Consistent with this hypothesis, it was observed that P16 significantly inhibited RBC hemolysis induced by group 1 A(H1N1) virus at pH 5.0 condition (Fig. 3E). Furthermore, it was confirmed that P16 blocked group 2 A(H7N7)-HA and FluB virus mediated cell fusion triggered by the low pH in 293T cells and MDCK cells. The low pH-treated cells without P16 showed >2-fold larger size than the normal cells treated by pH 7.4. These results indicated that P16 could broadly bind to stem region to block the low pH-induced HA conformational change (22). Finally, it was demonstrated that basic P16 inhibited endosomal acidification similar to the effect of bafilomycin A1 (16) in live cells. These results indicated that P16 could have dual functions which not only blocked HA fusion by binding but also inhibited endosomal acidification to interfere viral entry through the endocytic pathway.

### P16 inhibited SARS-CoV-2 by interfering with endosomal pH

The broad-spectrum antiviral activity of P16 was not restricted to influenza A and B viruses. P16 significantly inhibited SARS-CoV-2 infection with IC₅₀ of 2.9 µg ml⁻¹ for SARS-CoV-2 HKU001a, 3.0 µg ml⁻¹ for SARS-CoV-2 B.1.1.63 and 3.9 µg ml⁻¹ for SARS-CoV-2 B.1.617.2 as measured by plaque reduction assay (Fig. 5A). The antiviral activity of P16 mainly relied on targeting virus before viral entry (Fig. 5B) but not targeting cells when cells were treated before and after viral infection (Fig. 5C). P16 did not significantly inhibit viral release and viral attachment, but inhibited spike-ACE2 mediated cell fusion in 293T cells. These indicated that P16 blocked SARS-CoV-2 fusion with target cells. P16 and U5 bound to S protein of SARS-CoV-2 (Fig. 5D) and U5 reduced P16 binding to S protein, but not P9R binding to S protein (Fig. 5D), which indicated the competitive binding between U5 and P16 on S protein. However, U5 showed significantly weaker antiviral activity against SARS-CoV-2 infection than that of P16 (Fig. 5E), and did not inhibit spike-ACE2 mediated fusion. These results indicated that the binding of U5 and P16 to S protein itself could not significantly block spike-ACE2 mediated fusion to inhibit SARS-CoV-2 infection. The enhanced fusion inhibition of P16 on SARS-CoV-2 could be attributed to the inhibition of P16 on endosomal acidification, similar with the fusion inhibition of bafilomycin A1. It was further identified that P16 could not inhibit SARS-CoV-2 replication in Calu-3 cells, which was consistent to the activity of P16 against SARS-CoV-2 by inhibiting endosomal acidification. This is same as chloroquine which cannot inhibit SARS-CoV-2 replication in Calu-3 cells (24) because chloroquine inhibited endosomal acidification but not TMPRSS2 activity.

### Endosomal acidification inhibitors against viruses in vivo

To evaluate the antiviral efficacy of P16 *in vivo,* mice were challenged with A(H1N1)pdm09 virus. P16 significantly decreased lung viral load by 12-fold (Fig. 4C) and improved the survival of challenged mice by 80% (Fig. 4A). The protection conferred by P16 on infected mice was similar to that of zanamivir (Fig. 4C). Considering the drug resistant problems of anti-influenza drugs, it was tested whether the drug-resistant mutants of A(H1N1) virus could emerge when A(H1N1) virus was cultured in the presence of P16 (Fig. 4D). P16 efficiently inhibited the replication of passaged virus (P15 and P20), with similar efficiency against the virus without any passage (P0) (Fig. 4E).

Interestingly, intranasal inoculation of P16 significantly inhibited 14-fold SARS-CoV-2 replication in hamster lungs (Fig. 6A), which indicated that blocking endosomal acidification could inhibit SARS-CoV-2 replication in vivo even though P16 did not inhibit TMPRSS2-mediated infection of SARS-CoV-2 in Calu-3 cells. Thus, it was also demonstrated that intranasal administration of endosomal acidification inhibitor chloroquine (2 mg kg⁻¹) could inhibit 2-fold SARS-CoV-2 replication in hamster lungs (Fig. 6A). It was further demonstrated that intranasal administration of chloroquine could also inhibit 3.5-fold A(H1N1) virus replication in mice (Fig. 6B) when compared with mock, which was also less potent than that of P16 (12-fold) against A(H1N1) virus. To understand the highly significant antiviral effect of chloroquine against influenza virus and coronavirus in cell culture, but poor treatment effect in vivo, it was identified that chloroquine could not provide 1-day prophylactic activity inhibiting SARS-CoV and A(H1N1) virus replication in BALB/c mice, even though chloroquine has a long half life time (40). This indicated that the accumulation of chloroquine in endosomes but not in serum and cytosolic environment of lungs (39) did not provide the antiviral activity against pH-dependent viruses in vivo. Chloroquine could effectively inhibit viral replication when virus was treated with chloroquine during viral infection (Fig. 6C). However, treating cells with chloroquine before or after SARS-COV-2 infection, which did not effectively let chloroquine enter cells with virus together, showed significantly less antiviral activity than treating cells during viral infection (Fig. 6C). Thus, it was realized that chloroquine can exhibit antiviral activity in vivo if lung cells are constantly exposed to chloroquine with effective concentration against pH-dependent viruses. It was demonstrated that one dose of chloroquine could inhibit (3-5)-fold SARS-CoV and A(H1N1) virus replication in BALB/c mice when chloroquine was intranasally administrated into mouse lungs at the time before viral inoculation.

Most importantly, it was demonstrated that atomization inhalation of chloroquine could inhibit SARS-CoV and A(H1N1) virus replication in mice (Fig. 6D-6E), which showed that atomization inhalation of chloroquine can be used in clinical treatment for coronavirus patients and influenza patients. These in vivo data show that the endosomal acidification inhibitor chloroquine, which could not inhibit coronavirus or influenza virus replication in vivo when administrated by oral inoculation or intraperitoneal injection (29, 30, 39, 41), can effectively inhibit coronavirus and influenza virus replication in humans when chloroquine is administrated to lungs by intranasal routes, thus providing an effective chance of keeping lung cells in the bath of chloroquine environment. Thinking about the TMPRSS2-mediated entry pathway of SARS-CoV-2 in humans, it was further demonstrated that chloroquine could significantly inhibit SARS-CoV-2 replication in ex vivo human lung tissues which were kept in the bath of chloroquine (Fig. 6F). Overall, it was demonstrated that endosomal acidification inhibitors (P16 and chloroquine) could significantly inhibit SARS-CoV-2, SARS-CoV and influenza A(H1N1) virus replication in vivo through intranasal administration when lung cells could be bathed in chloroquine with effective concentration.

### Discussion

In this study, it was identified that endosomal acidification inhibitors (peptidic P16 and chemical chloroquine) could suppress influenza virus and coronavirus infection in vitro and in vivo. Dual-functional P16 could inhibit influenza virus by blocking influenza HA conformation change and inhibiting endosomal acidification to block pH-dependent influenza and coronavirus fusion. Importantly, it was demonstrated that both endosomal acidification inhibitors (P16 and chloroquine) could effectively inhibit A(H1N1) and SARS-CoV-2 replication in mice and hamsters. Chloroquine could significantly inhibit SARS-CoV-2 replication in ex vivo human lung tissues, which indicated that chloroquine could significantly inhibit SARS-CoV-2 replication in human lungs when chloroquine is delivered into lungs with effective concentration.

Broad-spectrum antivirals, like peptides binding to HA stem region, have been found to inhibit group 1 influenza A viruses (13, 22). However, the stem region is not very conserved in influenza virus (23), which may pose a challenge to find an universal antiviral against influenza virus by targeting the HA stem region. The results show that peptidic P16 can inhibit HA-mediated cell fusion of group 1 A(H1N1), group 2 A(H7N7) and FluB viruses, indicating that an antiviral peptide could be a fusion inhibitor with broad-spectrum activities against both influenza A and B viruses. Moreover, basic P16 binds to viruses and inhibits influenza virus and SARS-CoV-2 by preventing endosomal acidification. Broad-spectrum antivirals, like chloroquine elevating the endosomal pH of host cells without binding to virus, had been identified to inhibit viruses in vitro and in vivo (36, 37). However, a number of
studies demonstrated the lack of antiviral activity of chloroquine in vivo (29, 39, 42). Here, it was identified that both endosomal acidification inhibitors (P16 and chloroquine) could inhibit A(H1N1) virus in mice and SARS-CoV-2 replication in hamsters when administrated through intranasal routes. It was demonstrated that treating mice with chloroquine before viral inoculation could effectively inhibit influenza and coronavirus replication in vivo. However, one-day prophylactic treatment with chloroquine could not show antiviral activity against influenza and coronavirus in mice. It was further illustrated that the effective way to use chloroquine against pH-dependent virus is to provide a high enough concentration of chloroquine when virus is entering cells. Thus, it is important to provide the effective chance of keeping lung cells in chloroquine environment, which can effectively inhibit viral RNA release by preventing virus-endosome acidification.

Because of the high mutation rates resulting antigenic drift, influenza vaccines could only provide 10~60% protection during the past decades. Since the discovery of neuraminidase inhibitors in 1990 (26), M2 inhibitors (27) and polymerase inhibitors (4) have been discovered. However, viruses resistant to these inhibitors quickly emerged even during the clinical trials (3, 4, 20). In addition, with the circulation of pandemic SARS-CoV-2, different mutants have been reported. Thus, antivirals with different mechanisms and broad-spectrum activities against viruses and having a low likelihood of inducing drug resistance are needed for treating influenza virus and coronavirus.

P16 derived from frog defensin could broadly inhibit influenza A and B viruses and SARS-CoV-2 with low possibility to cause influenza drug-resistant virus. No drug-resistant virus was found after 20 viral passages in the presence of P16. Interestingly and importantly, chloroquine, despite not showing effective antiviral activity in vivo (28, 29, 30, 41) when administrated through systemic routes in many studies, could target host endosomes to block pH-dependent viral infection and significantly inhibit influenza virus and coronavirus replication in mice and hamsters when administrated by intranasal routes. The host-targeting antiviral activity of chloroquine indicates that chloroquine is less likely to induce drug-resistant problem. Currently, the rapid RT-PCR test can identify patients in the early period of infection which allows atomization of these endosomal acidification inhibitors to treat SARS-CoV-2 patients. For late presenters when the peak of viral load has passed, antiviral drugs given by any routes are unlikely to improve the outcome. Immunomodulatory agents such as steroid may play a more important therapeutic role. In addition, chloroquine could suppress the expression of inflammation cytokines in human lungs (43). Thus, the topical administration of chloroquine by atomization inhalation in the early period of infection may not only enhance the antiviral efficacy in lungs but also reduce the potential cardiac side effects when compared with systemic administration by oral or intravenous injection. These data show that the endosomal acidification inhibitor chloroquine can be used as a broad-spectrum antiviral candidate to treat coronavirus, influenza or co-infection of coronavirus and influenza virus in clinical trials through atomization inhalation.

### References:

1. T. Horimoto, Y. Kawaoka, Influenza: lessons from past pandemics, warnings from current incidents. Nat Rev Microbiol 3, 591-600 (2005).
2. T. Watanabe, S. Watanabe, E. A. Maher, G. Neumann, Y. Kawaoka, Pandemic potential of avian influenza A (H7N9) viruses. Trends Microbiol 22, 623-631 (2014).
3. H. Zhao et al., Dual-functional peptide with defective interfering genes effectively protects mice against avian and seasonal influenza. Nat Commun 9, 2358 (2018).
4. F. G. Hayden et al., Baloxavir Marboxil for Uncomplicated Influenza in Adults and Adolescents. N Engl J Med 379, 913-923 (2018).
5. N. Chai et al., Two Escape Mechanisms of Influenza A Virus to a Broadly Neutralizing Stalk-Binding Antibody. PLoS Pathog 12, e1005702 (2016).
6. N. C. Wu et al., Different genetic barriers for resistance to HA stem antibodies in influenza H3 and H1 viruses. Science 368, 1335-1340 (2020).
7. X. Zheng et al., Co-infection of SARS-CoV-2 and Influenza virus in Early Stage of the COVID-19 Epidemic in Wuhan, China. J Infect 81, e128-e129 (2020).
8. E. Cuadrado-Payán et al., SARS-CoV-2 and influenza virus co-infection. Lancet 395, e84 (2020).
9. A. J. Zhang et al., Co-infection by severe acute respiratory syndrome coronavirus 2 and influenza A(H1N1)pdm09 virus enhances the severity of pneumonia in golden Syrian hamsters. Clin Infect Dis, (2020*).*
10. S. Xia et al., Inhibition of SARS-CoV-2 (previously 2019-nCoV) infection by a highly potent pan-coronavirus fusion inhibitor targeting its spike protein that harbors a high capacity to mediate membrane fusion. Cell Res 30, 343-355 (2020).
11. S. Xia et al., A pan-coronavirus fusion inhibitor targeting the HR1 domain of human coronavirus spike. Sci Adv 5, eaav4580 (2019).
12. C. Wang et al., De Novo Design of α-Helical Lipopeptides Targeting Viral Fusion Proteins: A Promising Strategy for Relatively Broad-Spectrum Antiviral Drug Discovery. J Med Chem 61, 8734-8745 (2018).
13. D. J. Holthausen et al., An Amphibian Host Defense Peptide Is Virucidal for Human H1 Hemagglutinin-Bearing Influenza Viruses. Immunity 46, 587-595 (2017).
14. E. Leikina et al., Carbohydrate-binding molecules inhibit viral fusion and entry by crosslinking membrane glycoproteins. Nat Immunol 6, 995-1001 (2005).
15. H. Zhao et al., A novel peptide with potent and broad-spectrum antiviral activities against multiple respiratory viruses. Sci Rep 6, 22008 (2016).
16. H. Zhao et al., A broad-spectrum virus- and host-targeting peptide against respiratory viruses including influenza virus and SARS-CoV-2. Nat Commun 11, 4252 (2020).
17. T. Koyama, D. Platt, L. Parida, Variant analysis of SARS-CoV-2 genomes. Bull World Health Organ 98, 495-504 (2020).
18. D. C. Brice, G. Diamond, Antiviral Activities of Human Host Defense Peptides. Curr Med Chem 27, 1420-1443 (2020).
19. J. Ding, Y. Y. Chou, T. L. Chang, Defensins in viral infections. J Innate Immun 1, 413-420 (2009).
20. R. W. Finberg et al., Phase 2b Study of Pimodivir (JNJ-63623872) as Monotherapy or in Combination With Oseltamivir for Treatment of Acute Uncomplicated Seasonal Influenza A: TOPAZ Trial. J Infect Dis 219, 1026-1034 (2019).
21. K. K. Lai et al., Identification of Novel Fusion Inhibitors of Influenza A Virus by Chemical Genetics. Journal of virology 90, 2690-2701 (2015).
22. R. U. Kadam et al., Potent peptidic fusion inhibitors of influenza virus. Science 358, 496-502 (2017).
23. J. Sui et al., Structural and functional bases for broad-spectrum neutralization of avian and human influenza A viruses. Nat Struct Mol Biol 16, 265-273 (2009).
24. M. Hoffmann et al., Chloroquine does not inhibit infection of human lung cells with SARS-CoV-2. Nature, (2020*).*
25. J. S. Wadia, R. V. Stan, S. F. Dowdy, Transducible TAT-HA fusogenic peptide enhances escape of TAT-fusion proteins after lipid raft macropinocytosis. Nat Med 10, 310-315 (2004).
26. S. Kubo, T. Tomozawa, M. Kakuta, A. Tokumitsu, M. Yamashita, Laninamivir prodrug CS-8958, a long-acting neuraminidase inhibitor, shows superior anti-influenza virus activity after a single administration. Antimicrob Agents Chemother 54, 1256-1264 (2010).
27. A. D. Balgi et al., Inhibitors of the influenza A virus M2 proton channel discovered using a high-throughput yeast growth restoration assay. PLoS One 8, e55271 (2013).
28. D. Falzarano et al., Lack of protection against ebola virus from chloroquine in mice and hamsters. Emerg Infect Dis 21, 1065-1067 (2015).
29. P. Maisonnasse et al., Hydroxychloroquine use against SARS-CoV-2 infection in non-human primates. Nature, (2020*).*
30. D. J. Vigerust, J. A. McCullers, Chloroquine is effective against influenza A virus in vitro but not in vivo. Influenza Other Respir Viruses 1, 189-192 (2007).
31. X. Wang et al., The anti-influenza virus drug, arbidol is an efficient inhibitor of SARS-CoV-2 in vitro. Cell Discov 6, 28 (2020).
32. N. Lian et al., Umifenovir treatment is not associated with improved outcomes in patients with coronavirus disease 2019: a retrospective study. Clin Microbiol Infect 26, 917-921 (2020).
33. F. Findlay, L. Proudfoot, C. Stevens, P. G. Barlow, Cationic host defense peptides; novel antimicrobial therapeutics against Category A pathogens and emerging infections. Pathog Glob Health 110, 137-147 (2016).
34. H. Zhao et al., Novel residues in the PA protein of avian influenza H7N7 virus affect virulence in mammalian hosts. Virology 498, 1-8 (2016).
35. B. J. Zheng et al., Delayed antiviral plus immunomodulator treatment still reduces mortality in mice infected by high inoculum of influenza A/H5N1 virus. Proc Natl Acad Sci U S A 105, 8091-8096 (2008).
36. Keyaerts et al., Antiviral activity of chloroquine against human coronavirus OC43 infection in newborn mice. Antimicrob Agents Chemother 53, 3416-3421 (2009).
37. Y. Yan et al., Anti-malaria drug chloroquine is highly effective in treating avian influenza A H5N1 virus infection in an animal model. Cell Res 23, 300-302 (2013).
38. M. Huang, et al., Preliminary evidence from a multicenter prospective observational study of the safety and efficacy of chloroquine for the treatment of COVID-19. Nat Sci Rev. (2020).
39. S. J. F. Kaptein et al., Favipiravir at high doses has potent antiviral activity in SARS-CoV-2-infected hamsters, whereas hydroxychloroquine lacks activity. Proc Natl Acad Sci U S A 117, 26955-26965 (2020).
40. H. A. Karunajeewa et al., Pharmacokinetics of chloroquine and monodesethylchloroquine in pregnancy. Antimicrob Agents Chemother 54, 1186-1192 (2010).
41. H. Zhao et al., Cross-linking peptide and reporpused drugs inhibit both entry pathways of SARS-CoV-2. Nat Commun. Accepted in Jan, 2021.
42. N. I. Paton et al., Chloroquine for influenza prevention: a randomised, double-blind, placebo controlled trial. Lancet Infect Dis 11, 677-683 (2011).
43. S. Grassin-Delyle, et al., Chloroquine inhibits the release of inflammatory cytokines by human lung explants. Clin Infect Dis. 71:2265 (2020).
44. Chu, H. et al. Comparative Replication and Immune Activation Profiles of SARS-CoV-2 and SARS-CoV in Human Lungs: An Ex Vivo Study with Implications for the Pathogenesis of COVID-19. Clin Infect Dis, (2020*).*

It must be noted that as used herein and in the appended claims, the singular forms "a", "an", and "the" include plural reference unless the context clearly dictates otherwise.

Throughout the description and claims of this specification, the word "comprise" and variations of the word, such as "comprising" and "comprises," means "including but not limited to," and is not intended to exclude, for example, other additives, components, integers or steps.

"Optional" or "optionally" means that the subsequently described event, circumstance, or material may or may not occur or be present, and that the description includes instances where the event, circumstance, or material occurs or is present and instances where it does not occur or is not present.

Unless the context clearly indicates otherwise, use of the word "can" indicates an option or capability of the object or condition referred to. Generally, use of "can" in this way is meant to positively state the option or capability while also leaving open that the option or capability could be absent in other forms or embodiments of the object or condition referred to. Unless the context clearly indicates otherwise, use of the word "may" indicates an option or capability of the object or condition referred to. Generally, use of "may" in this way is meant to positively state the option or capability while also leaving open that the option or capability could be absent in other forms or embodiments of the object or condition referred to. Unless the context clearly indicates otherwise, use of "may" herein does not refer to an unknown or doubtful feature of an object or condition.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. It should be understood that all of the individual values and sub-ranges of values contained within an explicitly disclosed range are also specifically contemplated and should be considered disclosed unless the context specifically indicates otherwise. Finally, it should be understood that all ranges refer both to the recited range as a range and as a collection of individual numbers from and including the first endpoint to and including the second endpoint. In the latter case, it should be understood that any of the individual numbers can be selected as one form of the quantity, value, or feature to which the range refers. In this way, a range describes a set of numbers or values from and including the first endpoint to and including the second endpoint from which a single member of the set (i.e. a single number) can be selected as the quantity, value, or feature to which the range refers. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described.

Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinency of the cited documents. It will be clearly understood that, although a number of publications are referred to herein, such reference does not constitute an admission that any of these documents forms part of the common general knowledge in the art.

## Claims

1. An antiviral agent comprising one or more copies of P16 comprising the amino acid sequence of RGAHIKGRWKSRCHRF (SEQ ID NO:1).

2. The antiviral agent of claim 1, wherein the P16 has a net positive charge of at least 1, 2, 3, 4, 5 or 6; optionally
wherein the P16 has a net positive charge of at least 6; further optionally
wherein the P16 has a net positive charge of about 6.1.

3. The antiviral agent of any one of claims 1-2, wherein upon contact with a virus *in vitro,* the P16 binds to the virus, reduces or prevents fusion of the virus with target cells, inhibits endosomal acidification of target cells, reduces or prevents cell entry of the virus, reduces or prevents infection of target cells by the virus, reduces or prevents replication of the virus, or combinations thereof.

4. The antiviral agent of any one of claims 1-3 consisting of P16 (SEQ ID NO: 1).

5. A composition comprising a therapeutically effective amount of the antiviral agent of any one of claims 1-4 and a pharmaceutically acceptable carrier.

6. The composition of claim 5, wherein the composition is a unit dosage form; optionally
wherein the unit dosage form is selected from the group consisting of a table or capsule; or
wherein the unit dosage form is an injectable, wherein the composition further comprises a pharmaceutically acceptable carrier for injection to a human; or
wherein the composition is in a form suitable for intranasal or pulmonary delivery.

7. An antiviral agent according to any one of claims 1-4 or a composition according to any one of claims 6 to 7 for use in a method of treating a viral infection in a subject in need thereof, the method comprising administering an effective amount of the antiviral agent or the composition, to the subject.

8. The antiviral agent or the composition for use according to claim 7, wherein the infection is caused by a respiratory virus; optionally
wherein the infection is caused by a pH-dependent virus that requires endosomal acidification for virus-host membrane fusion.

9. The antiviral agent or the composition for use according to any one of claims 7-8, wherein the composition is administered parenterally or orally; or
wherein the composition is administered intranasally, or by pulmonary administration.

10. The antiviral agent or the composition for use according to any one of claims 7-9, wherein the infection is caused by a virus selected from zika virus, enterovirus-A7, ebola virus, influenza A virus, influenza B virus, SARS-CoV-2, SARS-CoV, MERS-CoV, the A(H1N1)pdm09 virus, and the non-enveloped rhinovirus; optionally
wherein the influenza virus is selected from influenza A(H7N9), influenza A(H7N7), influenza A(HSN1), influenza A(H1N1), and influenza A(H3N2); further optionally
wherein the subject is human.

11. The antiviral agent or the composition for use according to any one of claims 7-10, wherein the antiviral agent or composition is administered in an effective amount to reduce one or more symptoms of disease, disorder, or illness associated with virus; optionally
wherein the symptoms include fever, congestion in the nasal sinuses and/or lungs, runny or stuffy nose, cough, sneezing, sore throat, body aches, fatigue, shortness of breath, chest tightness, wheezing when exhaling, chills, muscle aches, headache, diarrhea, tiredness, nausea, vomiting, and combinations thereof; further optionally
wherein viral replication in the subject is inhibited.

12. The antiviral agent or the composition for use according to any one of claims 7-11 further comprising administering an effective amount of chloroquine to the subject, wherein the chloroquine is not administered orally; optionally
wherein the chloroquine is administered intranasally or by pulmonary administration.

13. The antiviral agent or the composition for use according to claim 12, wherein the chloroquine is in an inhalable form or formulation, optionally wherein the chloroquine is administered via intranasal inoculation or atomization inhalation.

## Patentansprüche

1. Antivirales Mittel, umfassend eine oder mehrere Kopien von P16, umfassend die Aminosäuresequenz von RGAHIKGRWKSRCHRF (SEQ ID NO:1).

2. Antivirales Mittel nach Anspruch 1, wobei das P16 eine positive Nettoladung von zumindest 1, 2, 3, 4, 5 oder 6 aufweist; optional
wobei das P16 eine positive Nettoladung von zumindest 6 aufweist; ferner optional
wobei das P16 eine positive Nettoladung von etwa 6,1 aufweist.

3. Antivirales Mittel nach einem der Ansprüche 1-2, wobei bei Kontakt mit einem Virus *in vitro* das P16 an das Virus bindet, Fusion des Virus mit Zielzellen reduziert oder verhindert, endosomale Ansäuerung von Zielzellen hemmt, Zelleintritt des Virus reduziert oder verhindert, Infektion von Zielzellen durch das Virus reduziert oder verhindert, Replikation des Virus reduziert oder verhindert oder Kombinationen davon.

4. Antivirales Mittel nach einem der Ansprüche 1-3, bestehend aus P16 (SEQ ID NO:1).

5. Zusammensetzung, umfassend eine therapeutisch wirksame Menge des antiviralen Mittels nach einem der Ansprüche 1-4 und einen pharmazeutisch verträglichen Träger.

6. Zusammensetzung nach Anspruch 5, wobei die Zusammensetzung eine Einheitsdosierungsform ist; optional
wobei die Einheitsdosierungsform ausgewählt ist aus der Gruppe bestehend aus einer Tabelle oder Kapsel; oder
wobei die Einheitsdosierungsform injizierbar ist, wobei die Zusammensetzung ferner einen pharmazeutisch verträglichen Träger zur Injektion in einen Menschen umfasst; oder
wobei die Zusammensetzung in einer Form ist, die für intranasale oder pulmonale Abgabe geeignet ist.

7. Antivirales Mittel nach einem der Ansprüche 1-4 oder Zusammensetzung nach einem der Ansprüche 6 bis 7 zur Verwendung in einem Verfahren zum Behandeln einer viralen Infektion bei einem Subjekt, das dessen bedarf, wobei das Verfahren Verabreichen einer wirksamen Menge des antiviralen Mittels oder der Zusammensetzung an das Subjekt umfasst.

8. Antivirales Mittel oder Zusammensetzung zur Verwendung nach Anspruch 7, wobei die Infektion durch ein Atemwegsvirus bewirkt wird; optional
wobei die Infektion durch ein pH-abhängiges Virus bewirkt wird, das endosomale Ansäuerung für Virus-Wirt-Membranfusion erfordert.

9. Antivirales Mittel oder Zusammensetzung zur Verwendung nach einem der Ansprüche 7-8, wobei die Zusammensetzung parenteral oder oral verabreicht wird; oder
wobei die Zusammensetzung intranasal oder durch pulmonale Verabreichung verabreicht wird.

10. Antivirales Mittel oder Zusammensetzung zur Verwendung nach einem der Ansprüche 7-9, wobei die Infektion durch ein Virus ausgewählt aus Zika-Virus, Enterovirus-A7, Ebola-Virus, Influenza-A-Virus, Influenza-B-Virus, SARS-CoV-2, SARS-CoV, MERS-CoV, dem A(H1N1)pdm09-Virus und dem nicht umhüllten Rhinovirus bewirkt wird; optional
wobei das Influenzavirus ausgewählt ist aus Influenza A(H7N9), Influenza A(H7N7), Influenza A(H5N1), Influenza A(H1N1) und Influenza A(H3N2); ferner optional
wobei das Subjekt menschlich ist.

11. Antivirales Mittel oder Zusammensetzung zur Verwendung nach einem der Ansprüche 7-10, wobei das antivirale Mittel oder die Zusammensetzung in einer wirksamen Menge verabreicht wird, um ein oder mehrere Symptome von Krankheit, Störung oder Erkrankung assoziiert mit Virus zu reduzieren; optional
wobei die Symptome Fieber, Verstopfung in den Nasennebenhöhlen und/oder Lungen, laufende oder verstopfte Nase, Husten, Niesen, Halsschmerzen, Körperschmerzen, Müdigkeit, Kurzatmigkeit, Engegefühl in der Brust, Keuchen beim Ausatmen, Schüttelfrost, Muskelschmerzen, Kopfschmerzen, Durchfall, Müdigkeit, Übelkeit, Erbrechen und Kombinationen davon beinhalten; ferner optional
wobei virale Replikation bei dem Subjekt gehemmt wird.

12. Antivirales Mittel oder Zusammensetzung zur Verwendung nach einem der Ansprüche 7-11, ferner umfassend Verabreichen einer wirksamen Menge an Chloroquin an das Subjekt, wobei das Chloroquin nicht oral verabreicht wird; optional
wobei das Chloroquin intranasal oder durch pulmonale Verabreichung verabreicht wird.

13. Antivirales Mittel oder Zusammensetzung zur Verwendung nach Anspruch 12, wobei das Chloroquin in einer inhalierbaren Form oder Formulierung ist, optional wobei das Chloroquin über intranasale Inokulation oder Zerstäubungsinhalation verabreicht wird.

## Revendications

1. Agent antiviral comprenant une ou plusieurs copies de P16 comprenant la séquence d'acides aminés de RGAHIKGRWKSRCHRF (SEQ ID N° : 1).

2. Agent antiviral de la revendication 1, dans lequel le P16 possède une charge nette positive d'au moins 1, 2, 3, 4, 5 ou 6 ; éventuellement
dans lequel le P16 possède une charge nette positive d'au moins 6 ; en outre, éventuellement
dans lequel le P16 possède une charge nette positive d'environ 6,1.

3. Agent antiviral de l'une quelconque des revendications 1 à 2, dans lequel, lors du contact avec un virus *in vitro,* le P16 se lie au virus, réduit ou empêche la fusion du virus avec des cellules cibles, inhibe l'acidification endosomale de cellules cibles, réduit ou empêche l'entrée du virus dans les cellules, réduit ou empêche l'infection de cellules cibles par le virus, réduit ou empêche la réplication du virus, ou des combinaisons de ceux-ci.

4. Agent antiviral de l'une quelconque des revendications 1 à 3, constitué de P16 (SEQ ID N° : 1).

5. Composition comprenant une quantité thérapeutiquement efficace de l'agent antiviral de l'une quelconque des revendications 1 à 4 et un support acceptable pharmaceutiquement.

6. Composition de la revendication 5, ladite composition étant une forme posologique unitaire ; éventuellement
ladite forme posologique unitaire étant choisie dans le groupe constitué par une table ou une capsule ; ou
ladite forme posologique unitaire étant injectable, ladite composition comprenant en outre un support acceptable pharmaceutiquement pour injection à un humain ; ou
ladite composition se présentant sous une forme appropriée pour une administration intranasale ou pulmonaire.

7. Agent antiviral selon l'une quelconque des revendications 1 à 4 ou composition selon l'une quelconque des revendications 6 à 7, destiné(e) à être utilisé(e) dans un procédé de traitement d'une infection virale chez un sujet en ayant besoin, le procédé comprenant l'administration d'une quantité efficace de l'agent antiviral ou de la composition, au sujet.

8. Agent antiviral ou composition destiné(e) à être utilisé(e) selon la revendication 7, ladite infection étant causée par un virus respiratoire ; éventuellement
ladite infection étant causée par un virus dépendant du pH qui nécessite une acidification endosomale pour la fusion de la membrane virus-hôte.

9. Agent antiviral ou composition destiné(e) à être utilisé(e) selon l'une quelconque des revendications 7 à 8, ladite composition étant administrée par voie parentérale ou orale ; ou
ladite composition étant administrée par voie intranasale ou par voie pulmonaire.

10. Agent antiviral ou composition destiné(e) à être utilisé(e) selon l'une quelconque des revendications 7 à 9, ladite infection étant causée par un virus choisi parmi le virus zika, l'entérovirus A7, le virus Ebola, le virus de la grippe A, le virus de la grippe B, le SARS-CoV-2, le SARS-CoV, le MERS-CoV, le virus A(H1N1)pdm09 et le rhinovirus non enveloppé ; éventuellement
ledit virus de la grippe étant choisi parmi la grippe A(H7N9), la grippe A(H7N7), la grippe A(H5N1), la grippe A(H1N1) et la grippe A(H3N2) ; en outre, éventuellement
ledit sujet étant humain.

11. Agent antiviral ou composition destiné(e) à être utilisé(e) selon l'une quelconque des revendications 7 à 10, ledit agent antiviral ou ladite composition étant administré(e) en quantité efficace pour réduire un ou plusieurs symptômes d'une maladie, d'un trouble ou d'une pathologie associés au virus ; éventuellement
lesdits symptômes comprenant la fièvre, la congestion des sinus nasaux et/ou des poumons, le nez qui coule ou bouché, la toux, les éternuements, les maux de gorge, les douleurs corporelles, la fatigue, l'essoufflement, la gêne respiratoire, la respiration sifflante à l'expiration, les frissons, les douleurs musculaires, les maux de tête, la diarrhée, l'épuisement, les nausées, les vomissements et des combinaisons de ceux-ci ; en outre, éventuellement,
ladite réplication virale chez le sujet étant inhibée.

12. Agent antiviral ou composition destiné(e) à être utilisé(e) selon l'une quelconque des revendications 7 à 11, comprenant en outre l'administration d'une quantité efficace de chloroquine au sujet, ladite chloroquine n'étant pas administrée par voie orale ; éventuellement
ladite chloroquine étant administrée par voie intranasale ou par voie pulmonaire.

13. Agent antiviral ou composition destiné(e) à être utilisé(e) selon la revendication 12, ladite chloroquine se présentant sous une forme ou une formulation inhalable, éventuellement ladite chloroquine étant administrée par inoculation intranasale ou inhalation par atomisation.
